# EUROPEAN PATENT APPLICATION

(11) **EP 1 728 465 A1**
(43) Date of publication of application: **06.12.2006**
(21) Application number: 05720919.9
(22) Date of filing: 16.03.2005
(51) Int. Cl.: A61B 1/12

(54) **ENDOSCOPE WASHING AND STERILIZING SYSTEM, ENDOSCOPE WASHING AND STERILIZING DEVICE AND ENDOSCOPE**

(30) Priority: 22.03.2004 JP 2004083663; 29.09.2004 JP 2004284971
(71) Applicant: Olympus Corporation, Tokyo 151-0072 (JP)
(72) Inventor: Suzuki, Eiri c/o Olympus Intellectual, Hachioji-shi, Tokyo 192-8512 (JP); NOGUCHI, Toshiaki c/o Olympus Intellectual, Hachioji-shi, Tokyo 192-8512 (JP); GOCHO, Masanori c/o Olympus Intellectual, Hachioji-shi, Tokyo 192-8512 (JP); HASEGAWA, Hitoshi c/o Olympus Intellectual, Hachioji-shi, Tokyo 192-8512 (JP); KUROSHIMA, Hisashi c/o Olympus Intellectual, Hachioji-shi, Tokyo 192-8512 (JP)
(74) Representative: von Hellfeld, Axel
(86) International application number: PCT/JP2005/004665
(87) International publication number: WO 2005/089633

(57) **Abstract**

An endoscope washer system comprises: an endoscope having a channel positioned on the inside thereof and comprising an insertion portion and an operating unit; and an endoscope washer device comprising a fluid supplying channel capable of moving between a retreated position distanced from the channel of the endoscope set in a predetermined position within the cleaning sink and a first utilizing position, to which the fluid supplying channel advances, toward the channel side from this retreated position, and makes connection to the channel of the endoscope, and a fluid channel drive mechanism that recognizes that the endoscope is positioned at the predetermined position within the cleaning sink, and automatically moves the fluid supplying channel from the retreated position to the first utilizing position.

## Description

### Technical Field

The present invention relates to an endoscope that has an internal channel, an endoscope washer device that washes and sterilizes this endoscope, and an endoscope washer system that includes the endoscope and the endoscope washer device.

### Background Art

In recent years, the endoscope has been widely used in medical fields and in industrial fields. The endoscope that is used in the medical fields has a long thin insertion portion that is inserted into the body cavity. This endoscope wherein the insertion portion is inserted into the body cavity has treatment equipment that is inserted into an insertion channel as necessary for observation of organs or for various types of treatment.

In particular, endoscopes for the medical field are used by insertion into the body cavity for the purpose of examination and treatment, and therefore cleaning and sterilizing of the endoscope is necessary. In the case of cleaning and sterilizing of this endoscope, an endoscope washer device may be used.

The endoscope is set in a cleaning sink of the endoscope washer device, and is subjected to cleaning and sterilization, rinsed, and dried.

Further, the inside of the endoscope has multiple channels such as a water and air sending channel and a forceps opening. It is necessary to pass sufficient abluent and sterilizing fluid through these channels, and ensure cleaning and sterilization.

Such an endoscope washer device that performs cleaning and sterilization of endoscopes has been proposed in Japanese Unexamined Patent Application Publication No. 09-253029, for example.

However, the cleaning and sterilizing of the endoscope must be performed not only on the outer surface of the endoscope but also in the channels of the endoscope. Thus, when such an endoscope washer device as disclosed in Japanese Unexamined Patent Application Publication No. 09-253029 is used and an endoscope is provided in the cleaning sink thereof, it is required for the worker to connect multiple cleaning tubes to all the channels in the endoscope respectively.

In the connection work, one ends of the cleaning tubes must be connected to the connecting ports of the channels which open at the side of the endoscope respectively, and the other ends of the cleaning tubes must be connected to the cleaning tube connectors which are provided so as to correspond to the connecting ports of the channels respectively at the side of the endoscope washer device. In so doing, if the endoscope has many channels, it takes time to connect the multiple cleaning tubes thereto, so that the time needed for cleaning and sterilizing the endoscope is prolonged. As a result, the workability of the endoscope becomes lowered. This is a problem.

Furthermore, since the cleaning tubes are manually connected, if the number of the cleaning tubes that are to be connected increases, the connecting work must be done more carefully accordingly so as not to fail to connect any of them, and also the time is needed to check whether they are connected accurately.

Still furthermore, a predetermined strength for connection is required at the respective connecting parts between the various channels within the endoscope and the multiple cleaning tubes, and the connection thereof is left to the user.

Still furthermore, in the endoscope whose insertion portion is inserted in the body cavity of the patient, it is needed to have the outer surface of the insertion portion as well as the channels within the endoscope reliably cleaned and sterilized.

Hence, the present invention which has been achieved under the circumstances is to provide an endoscope washer system, an endoscope and an endoscope washer device making it possible to easily set the endoscope in a cleaning sink and reliably perform the cleaning and sterilizing of the various channels of the endoscope without connecting work by the user to connect the multiple cleaning tubes of the endoscope washer device to the various channels within the endoscope when performing the cleaning or sterilizing of the endoscope.

### Disclosure of Invention

### Means for Solving the Problem

The endoscope washer system according to the present invention including an endoscope and an endoscope washer device comprises: an endoscope having a channel positioned on the inside thereof and comprising an insertion portion and an operating unit; and an endoscope washer device comprising a fluid supplying channel capable of moving between a retreated position distanced from the channel of the endoscope set in a predetermined position within the cleaning sink and a first utilizing position, to which the fluid supplying channel advances, toward the channel side from this retreated position, and makes connection to the channel of the endoscope, and a fluid channel drive mechanism that recognizes that the endoscope is positioned at the predetermined position within the cleaning sink, and automatically moves the fluid supplying channel from the retreated position to the first utilizing position.

### Brief Description of the Drawings

Fig. 1 is a diagram illustrating a schematic configuration of an endoscope washer system relating to a first embodiment;
Fig. 2 is a perspective view illustrating the external view of the endoscope set in a cleaning sink of an endoscope washer device relating to the first embodiment;
Fig. 3 is a diagram illustrating the configuration of an endoscope relating to the first embodiment;
Fig. 4 is a partial perspective view of a state wherein the insertion portion is detached from the operating unit of the endoscope relating to the first embodiment;
Fig. 5 is a front view of the proximal-end face of the insertion portion of the endoscope relating to the first embodiment;
Fig. 6 is a front view of the tip face of the insertion portion of the endoscope relating to the first embodiment;
Fig. 7 is a partial cross-sectional view along the lengthwise axis of the endoscope in the state wherein the insertion portion and the operating unit of the endoscope relating to the first embodiment are linked;
Fig. 8 is a perspective view illustrating a channel joint portion of the endoscope washer device relating to the first embodiment;
Fig. 9 is a cross-sectional diagram illustrating the link between the insertion portion of the endoscope and the linking portion of the channel joint portion of the endoscope washer device relating to the first embodiment;
Fig. 10 is a cross-sectional diagram illustrating the link between the operating unit of the endoscope and the linking portion of the channel joint portion of the endoscope washer device relating to the first embodiment;
Fig. 11 is a diagram illustrating a guide groove formed on the periphery of the insertion portion of the endoscope;
Fig. 12 is a diagram illustrating the guide groove formed on the periphery of the insertion portion of the endoscope;
Fig. 13 is a diagram illustrating a guide hole formed on the periphery of the insertion portion of the endoscope;
Fig. 14 is a diagram illustrating a linking portion of the channel joint portion that is fixed to one wall face of the cleaning sink of the endoscope washer device;
Fig. 15 is a diagram illustrating notification means positioned in the insertion portion of the endoscope;
Fig. 16 is a partial configuration diagram illustrating an internal portion wherein the endoscope is set in the cleaning sink of the endoscope washer device relating to a second embodiment;
Fig. 17 is a diagram illustrating a cross-section of the channel cleaning nozzle of the endoscope washer device relating to the second embodiment;
Fig. 18 is a diagram illustrating a cross-section of a channel cleaning nozzle of the endoscope washer device relating to the second embodiment;
Fig. 19 is a configuration diagram of the endoscope washer device illustrating an internal portion wherein the endoscope is set in the cleaning sink of the endoscope washer device relating to a third embodiment;
Fig. 20 is a cross-sectional diagram illustrating the external portion of the cleaning sink wherein the endoscope is set in the cleaning sink of the endoscope washer device relating to the third embodiment; and
Fig. 21 is an explanatory diagram illustrating the endoscope having a positioning groove of the operating unit being held by the operating unit holding unit.

### Best Mode for Carrying Out the Invention

### [First Embodiment]

An endoscope washer system 300 according to the present embodiment, that includes an endoscope washer device 40 and an endoscope 1A, will be described below, based on the diagrams.

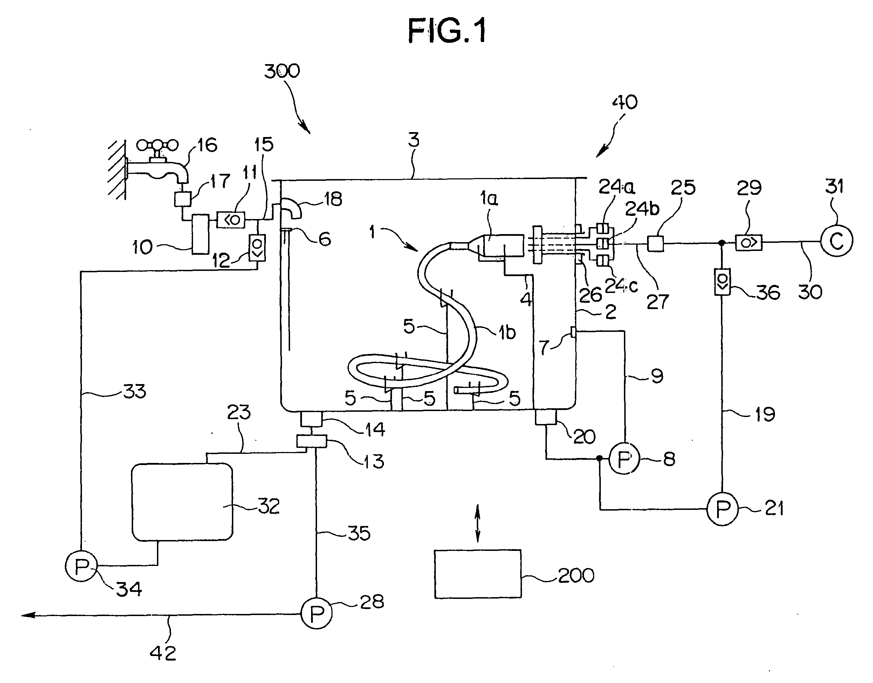

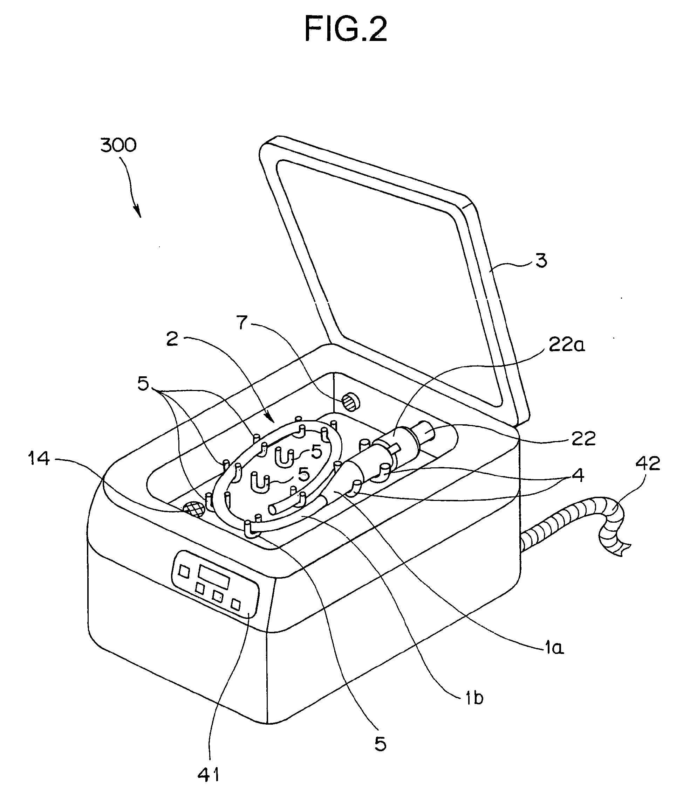

Fig. 1 is a diagram illustrating a schematic configuration of the endoscope washer system 300, and Fig. 2 is a perspective view illustrating the external view wherein an insertion portion 1 of the endoscope 1A is set in a cleaning sink 2 of the endoscope washer device 300.

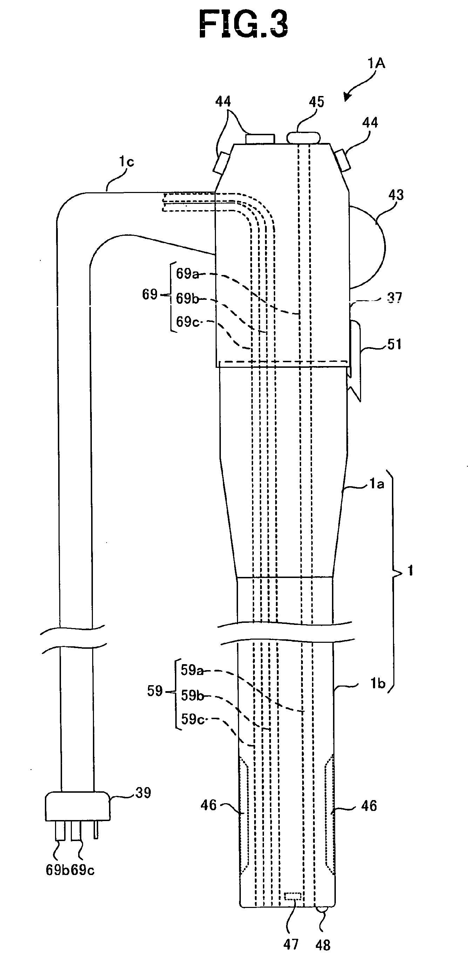

The endoscope washer system 300 includes an endoscope washer device 40, and the endoscope 1A which is to be cleaned and sterilized (see Fig. 3). Now, with the present embodiment, only an insertion portion 1a of the endoscope 1A is illustrated in Fig. 1.

The endoscope washer device 40 has the sink 2 for the purpose of cleaning or sterilizing of the insertion portion 1 of the endoscope 1A (hereafter referred to as "cleaning sink"), and a top cover 3 provided on the upper portion of this cleaning sink 2.

Further, the insertion portion 1 of the endoscope 1A is set in the cleaning sink 2 within the endoscope washer device 40. The insertion portion 1 is set so that the first insertion portion 1a and the second insertion portion 1b are to be held in a predetermined position within the cleaning sink 2 by a first holding member 4 and multiple second holding members 5.

The first holding member 4 has sensors (not illustrated), for example an optical sensor or a contact sensor and the like, that recognize and detect that the first insertion portion 1a of the insertion portion 1 is set.

A water level detecting sensor 6 that detects the water level of the circulating fluid that circulates throughout the endoscope washer device 40, and a nozzle 18 that is the injection opening for the dilution circulating fluid, are provided on one wall face of the inner side of the cleaning sink 2.

The water detecting sensor 6 has two liquid level detecting units for detecting that the liquid level of the circulating fluid stored within the cleaning sink 2 of the endoscope washer device 40 has reached at least two levels of different heights from the floor of the cleaning sink 2.

One of the liquid level detecting units (hereafter referred to as "first liquid level detecting unit") is provided on the floor side of the cleaning sink 2 so as to detect the predetermined water level of the circulating fluid at a lower level than the other liquid level detecting unit (hereafter referred to as "second liquid level detecting unit").

Further, the second liquid level detecting unit is provided so as to detect the predetermined water level of the circulating fluid wherein the insertion portion 1 that is set in the cleaning sink 2 is sufficiently submerged.

A spigot 16 on the outside of the endoscope washer device 40 for supplying the tap water as the dilution circulation fluid is connected with the nozzle 18 that is provided on the inner wall face of the upper side of the cleaning sink 2 so as to communicate via a water supply channel 15.

Along this water supply channel 15 is provided a water supply valve 17 that is an electromagnetic valve which supplies and stops tap water or the like, a water supply filter 10 that filters the tap water, and a check valve 11 that prevents the backflow of the circulating fluid, in that order.

A control signal for a control circuit 200 is provided in order for the water supply valve 17 to supply and stop the tap water, whereby the inner valve opens and closes. Further, the nozzle 18 is connected to a sterilizing fluid injection channel 33 of which one end is connected to the water supply channel 15. Further, the other end of the sterilizing fluid injection channel 33 is connected so as to communicate with a sterilizing fluid tank 32 wherein the sterilizing fluid is stored.

An amount of the sterilizing fluid sufficient to submerge the insertion portion 1 that is set in the cleaning sink 2 is stored within the sterilizing fluid tank 32. Now, this sterilizing fluid has a predetermined number of times of use effective for the later-described sterilizing process, and after this number of times of use is completed, the sterilizing fluid is replaced with new sterilizing fluid.

An injecting pump 34 and a check valve 12 are configured within the sterilizing fluid injection channel 33, in that order from the sterilizing fluid tank 32 side. The sterilizing fluid tank 32 is also connected to a chemical collecting channel 23.

This chemical collecting channel 23 is connected so as to communicate with a drain opening 14 provided on the bottom face of the cleaning sink 2. A switching valve 13 is provided on this chemical collecting channel 23, between the sterilizing fluid tank 32 and the drain opening 14. Further, the switching valve 13 is also connected to a draining channel 35.

The switching valve 13 allows communication between the cleaning sink 2 and the draining channel 35, and communication between the cleaning sink 2 and the chemical collecting channel 23, by switching an internal valve. Further, the switching valve 13 can close the internal valve so that neither the draining channel 35 nor the chemical collecting channel 23 can communicate with the cleaning sink 2.

A draining pump 28 suctions the circulating fluid of the cleaning sink 2 from the draining channel 35 side, and sends it to a later-described draining hose 42 (see Fig. 2) side. Thus, the circulating fluid that is stored in the cleaning sink 2 is discharged to the outside of the endoscope washer device 40.

The injecting pump 34, the switching valve 13, and the draining pump 28 are all electrically connected to the above described control circuit 200, which controls opening and closing, starting, stopping, and so forth.

Further, the cleaning sink 2 is provided with a circulating fluid suction opening 20 on the floor face thereof, and a circulating fluid discharge opening 7 on one wall face. The circulating fluid suction opening 20 and the circulating fluid discharge opening 7 are connected so as to communicate by a circulating fluid cleaning/sterilizing channel 9.

The circulating fluid cleaning/sterilizing channel 9 is provided with a circulating fluid cleaning/sterilizing pump 8. One end of a channel interior cleaning channel 19 is connected to the circulating fluid cleaning/sterilizing channel 9, which is between the circulating fluid cleaning/sterilizing pump 8 and the circulating fluid suction opening 20.

The other end of the channel interior cleaning channel 19 is connected so as to communicate with an air supply channel 30. Further, the channel interior cleaning channel 19 is provided with a channel interior cleaning pump 21 and a check valve 36 in that order from the connection with the circulating fluid cleaning/sterilizing channel 9.

One end of the air supply channel 30 is connected to a compressor 31, and the other end is connected to an electromagnetic opening/closing valve 25. Further, the air supply channel 30 is provided with a check valve 29 between the connecting unit that connects to the channel interior cleaning channel 19 and the compressor 31.

The electromagnetic opening/closing valve 25 is connected to a branch channel 27 that branches in three directions. The branch ends wherein the branch channel 27 has branched in three directions are connected to three electromagnetic opening/closing valves 24a, 24b, 24c that each have a flow rate sensor.

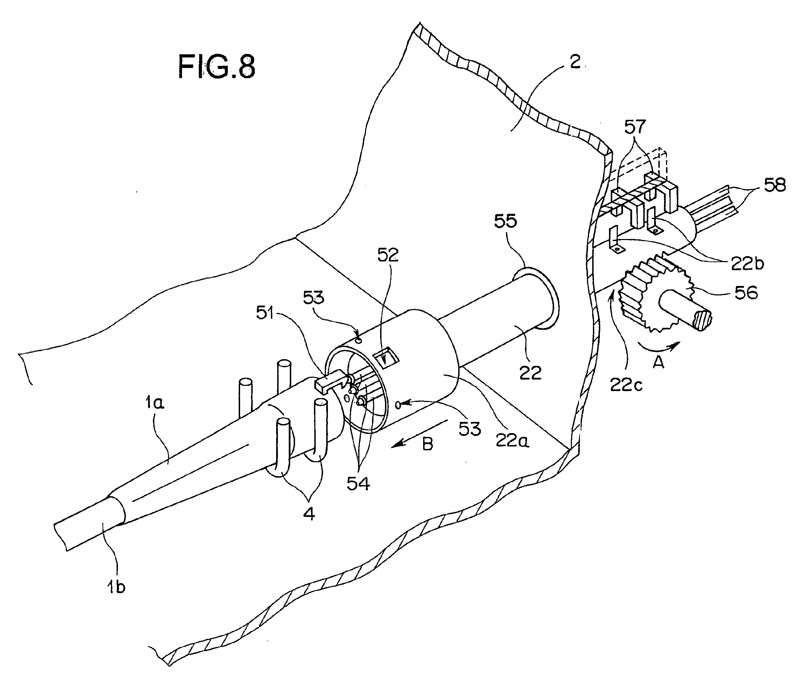

These three electromagnetic opening/closing valves are each connected to three fluid sending tubes 58 that communicate with the later-described channel cleaning nozzle 54 (see Fig. 8) of a channel joint portion 22.

The channel joint portion 22 passes through a hole that is provided in the side wall of the cleaning sink 2. This channel joint portion 22 is moved forward within the cleaning sink 2 or is retreated farther from the inner side thereof, by a later-described drive mechanism 26.

As illustrated in Fig. 2, the endoscope washer device 40 of the endoscope washer system 300 has an operating panel 41 on one side face of the exterior member, upon which are disposed various switches which start or stop various processes, and a display panel. This operating panel 41 is electrically connected to the control circuit 200 within the device, and supplies various instruction signals to the control circuit 200.

The control circuit 200 also supplies control signals to the various pumps and various electromagnetic valves of the endoscope washer device 40.

Further, a draining hose 42 is extended from main body of the endoscope washer device 40 for externally draining the internal circulating fluid.

A hole is provided on one inner wall face of the cleaning sink 2 of the endoscope washer device 40, and the channel joint portion 22 extends from this hole towards the first insertion portion 1a of the insertion portion 1 that is placed in the cleaning sink 2. A later-described sealing member 55 (see Fig. 8) is provided on this hole in the cleaning sink 2.

The insertion portion 1 of the endoscope 1A illustrated in Fig. 2 has the first and second inserting units 1b set so as to be secured by the first holding member 4 and the multiple second holding members 5 that are provided within the cleaning sink 2 of the endoscope washer device 40.

Further, the first insertion portion 1a is positioned in the first holding member 4 at a predetermined position.

The first insertion portion 1a of the insertion portion 1 is connected to the channel joint portion 22, and later-described multiple endoscope channels 59 of the insertion portion 1 and multiple channel cleaning nozzles 54 of the channel joint portion 22 communicate.

As described above, the first holding member 4 is provided with, for example, an optical sensor or the like that detects the first insertion portion 1a of the insertion portion 1 being set as predetermined. Now, the position wherein the multiple channel cleaning nozzles 54 and the multiple endoscope channels 59 are connected becomes the first utilizing position for each process of the endoscope washer device 40.

Here, the configuration of the endoscope 1A that is cleaned and sterilized by the endoscope washer device 40 according to the present embodiment will be described below, with reference to the drawings.

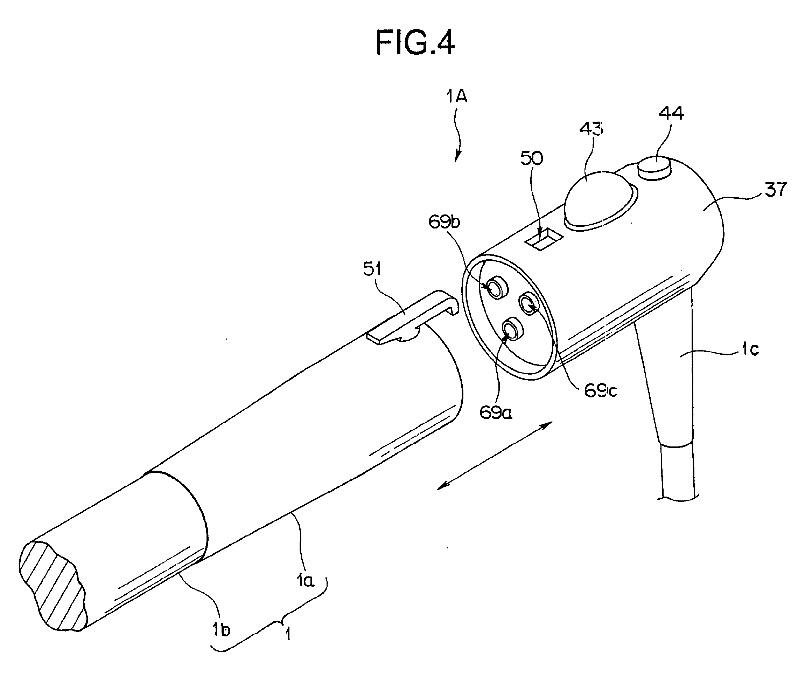

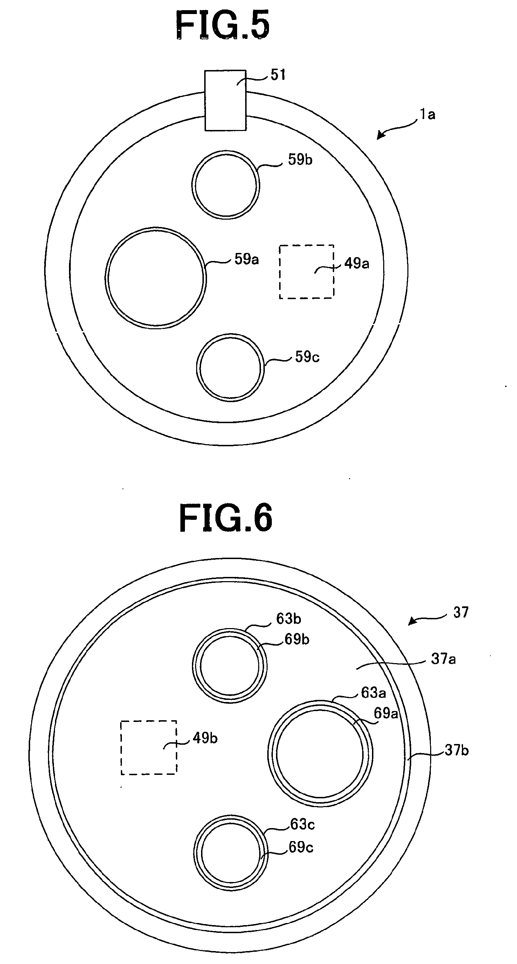

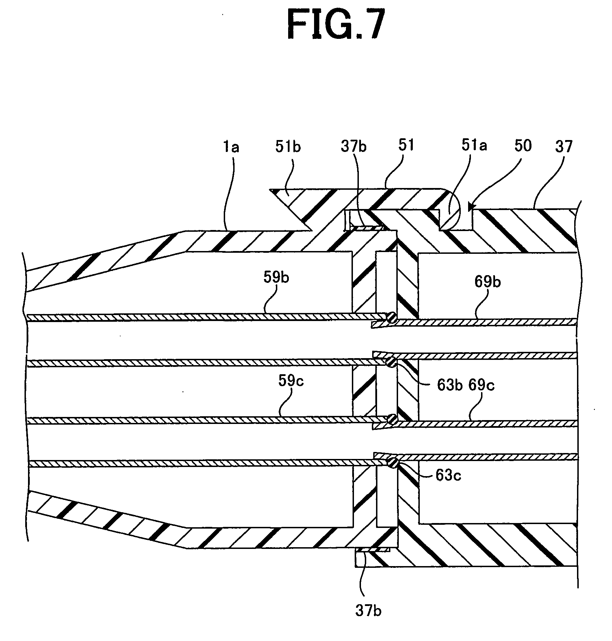

Fig. 3 is a diagram illustrating the configuration of the endoscope 1A, Fig. 4 is a partial plan view of the state wherein the insertion portion 1 is detached from the operating unit 37 of the endoscope 1A, Fig. 5 is a front view of the proximal-end face of the insertion portion 1, Fig. 6 is a front view of the tip face of the operating unit 37, and Fig. 7 is a partial cross-sectional view along the lengthwise axis of the endoscope 1A in the state wherein the insertion portion 1 and an operating unit 37 are linked.

As illustrated in Fig. 3, the endoscope 1A according to the present embodiment primarily comprises an insertion portion 1 that is long and flexible, the operating unit 37 that is connected to the proximal end of the insertion portion 1, and a universal cord 1c that extends from the side of the operating unit 37 and on which a connector unit 39 is provided on the extending end thereof.

The insertion portion 1 includes the first insertion portion 1a that serves as a base for bending protection, and a second insertion portion 1b that is inserted into the body cavity. A hook 51 which is a retaining portion is provided on the external portion of the proximal-end side of the first insertion portion 1a.

Further, the second insertion portion 1b is provided with an LED 48 that is an illuminating member on the tip face, an imaging element 47 on the interior of the tip portion such as a CCD which is an imaging means, and an angling member 46 made from EPAM (electro active polymer), that bends and moves the tip portion of the second insertion portion 1b along with the operation of a track ball 43 of a later-described operating unit 37, on the periphery of the tip portion.

Multiple endoscope channels 59 that pass through from the tip end to the proximal end are provided in the insertion portion 1. These endoscope channels 59 include a therapeutic instrument channel 59a wherein therapeutic instruments and so forth are passed through, an air and water sending channel 59b for sending air and sending water, and a suction channel 59c for suctioning.

Further, within the operating unit 37, various endoscope channels 69, i.e., a therapeutic instrument channel 69a, an air and water sending channel 69b, and a suction channel 69c, are provided. The therapeutic instrument channel 69a is open on one end, at the proximal end of the operating unit 37. Further, the air and water sending channel 69b and the suction channel 69c follow along the inside of the universal cord 1c or on the outside thereof, and one end thereof is open at the connector unit 39.

On the other hand, the other ends of the various endoscope channels 69 are open at the tip end of the operating unit 37. These endoscope channels 69 are freely detachable from the operating unit 37 and the universal cord 1c, and are discarded with each use of the endoscope 1A.

Now, the connections between the various endoscope channels 59 on the insertion portion 1 side and the various endoscope channels 69 of the operating unit 37 will be described below.

The operating unit 37 is provided with a cap 45 on the proximal end for fitting the opening of the therapeutic instrument channel 69a, and various buttons 44 on the proximal end to operate in the case of sending air, sending water, or suctioning.

The connector unit 39 of the universal cord 1c is connected to the control unit not illustrated. This control unit is an external equipment for performing various power supply, and supplies functions for suctioning, sending air, and sending water according to the operation of the various buttons 44 of the operating unit 37.

As illustrated in Fig. 4, the insertion portion 1 is such that the proximal-end portion of the first insertion portion 1a is detachable from the tip portion of the operating unit 37. The insertion portion 1 is linked to the operating unit 37 by means of retaining the clip section 51a on the tip portion of the hook 51 onto a hook hole 50 that is a retaining portion formed on the periphery of the operating unit 37.

Next, the proximal-end face of the insertion portion 1 and the tip face of the operating unit 37 will be described, based on Figs. 5 and 6.

As illustrated in Fig. 5, the tip face of the insertion portion 1 has the various protruding portions of the therapeutic instrument channel 59a, the air and water sending channel 59b, and the suction channel 59c, and an inserting-portion-side transmitting coil 49a wherein electric power from the operating unit 37 is supplied without contact by the operating unit transmitting coil 49b illustrated in Fig. 6, provided on the inside of the tip face.

Further, as illustrated in Fig. 6, the tip face of the operating unit 37 has the protruding portions of the therapeutic instrument channel 69a, the air and water sending channel 69b, and the suction channel 69c that each engage with the various channels 59a through 59c that are provided on the tip face of the insertion portion 1, and the above-described operating unit transmitting coil 49b is disposed.

O-rings 63a through 64c are each provided on the periphery of the therapeutic instrument channel 69a, the air and water sending channel 69b, and the suction channel 69c that are provided on the tip face of the operating unit 37.

Further, on the tip portion of the operating unit 37, a concave portion 37a is formed as a hole to which the proximal end of the insertion portion 1a of the insertion portion 1 is engaged. On the inner wall of the operating unit 37 that forms this concave portion 37a is provided a packing 37b. Now, the concave portion 37a of the operating unit 37 has approximately the same hole diameter as the outside diameter of the first insertion portion 1a.

Therefore, as illustrated in Fig. 7, the first insertion portion 1a adheres to the inner wall of the operating unit 37 wherein the periphery portion of the proximal-end portion forms the concave portion 37a, and is connected to the operating unit 37 so as to be airtight.

The protruding portions on the tip faces of the various channels 69a through 69c that have opening portions each protrude to the proximal-end face of the operating unit 37 so as to correspond with the various channels 59a through 59c of the first insertion portion 1. Further, the protruding portions of the various channels 59a through 59c have approximately the same external diameters as the inner diameters of the various channels 59a through 59c of the first insertion portion 1a.

The first insertion portion 1a is linked to the operating unit 37 with the position thereof being adjusted, so that the hook 51 can be engaged with the hook hole 50 of the operating unit 37. In other words, the hook 51 of the first insertion portion 1a and the hook hole 50 of the operating unit 37 have predetermined positions so that the protruding portions of the various channels 69a through 69c of the operating unit 37 are connected into the corresponding opening portions of the various channels 59a through 59c of the first insertion portion 1a.

As a result, improper connection of the various channels 59a through 59c of the insertion portion 1 and the various channels 69a through 69c of the operating unit 37 is prevented. Therefore, the therapeutic instrument channel 59a of the insertion portion 1 connects in a sure manner to the therapeutic instrument channel 69a of the operating unit 37. Further, the air and water sending channel 59b of the insertion portion 1 connects in a sure manner to the air and water sending channel 69b of the operating unit 37. Further, the suction channel 59c of the insertion portion 1 connects in a sure manner to the suction channel 69c of the operating unit 37.

Now, in the state wherein the first insertion portion 1a is connected to the operating unit 37, when the protruding portions of the various channels 69a through 69c of the operating unit 37 are connected into the opening portions of the various channels 59a through 59c of the first insertion portion 1a, the connection is made airtight by the O-rings 63a through 63c that are provided on the periphery.

The hook 51 has a clip section 51a wherein one portion of the tip portion thereof is chamfered. Further, the hook 51 has a pinching section 51b that is cut out in a sharp angle to the direction approximately perpendicular to the lengthwise direction of the proximal-end portion.

When force is placed on the pinching section 51b of the proximal-end portion in the downward direction in Fig. 7, the portion in contact with the first insertion portion 1a serves as a fulcrum such that the tip of the clip section 51a can be moved in the upward direction in Fig. 7.

Therefore, by placing force in the downward direction in Fig. 7 on the pinching section 51b of the hook 51 and moving the clip section 51a upwards in Fig. 7, the first insertion portion 1a is removed from the hook hole 50 of the operating unit 37. Then by being pulled apart from the operating unit 37, the first insertion portion 1a is detached from the operating unit 37.

On the other hand, in the case of linking the first insertion portion 1a to the operating unit 37, the first insertion portion 1a is pressed into the operating unit 37 while the clip section 51a is in the state of moving upwards in Fig. 7. Then by the force in the downward direction in Fig. 7 that was placed on the pinching section 51b of the hook 51 being removed, the clip section 51a moves in the downward direction in Fig. 7. Then by the clip section 51a of the hook 51 being engaged with the hook hole 50 of the operating unit 37, the first insertion portion 1a is linked to the operating unit 37.

Now, with the present embodiment, the hook 51 serving as a retaining portion is provided on the first insertion portion 1a of the insertion portion 1, and a hook hole 50 that is a retained portion is provided on the operating unit 37; however, the reverse can be arranged, in which a hook hole is provided on the first insertion portion 1a and a hook is provided on the operating unit 37.

Further, the inserting-portion-side transmitting coil 49a that is housed in the proximal-end portion of the first insertion portion 1a is positioned in the corresponding position to the operating unit transmitting coil 49b that is housed in the tip portion of the operating unit.

Further, magnets can be provided on the tip face of the operating unit 37 and the proximal-end face of the first insertion portion 1a, and the linking force of the first insertion portion 1a and the operating unit 37 can be strengthened.

Now, for the angling member 46 that allows bending movements of the tip portion of the second insertion portion 1b, operating signals by operation of the track ball 43 of the operating unit 37 is supplied to a reception antenna, not shown, that is housed in the first insertion portion 1a, by a transmission antenna, not shown, that is housed in the operating unit 37.

The insertion portion 1 of the endoscope 1A that is configured as described above is set into the cleaning sink 2 of the endoscope washer device 40, and is cleaned and sterilized.

Next, the channel joint portion 22 of the endoscope washer device 40 will be described in detail.

Fig. 8 is a perspective view illustrating a channel joint portion 22 that links to the first insertion portion 1a of the insertion portion 1.

As illustrated in Fig. 8, the channel joint portion 22 is in a channel form wherein the fluid sending tube 58 passes through from the proximal end. This channel joint portion 22 has a linking portion 22a on the tip side that links so as to be detachably linked with the first insertion portion 1a of the insertion portion 1, and two convex portions 22b that are sensor detecting members that are provided on the surface of the proximal end by fixing members such as screws.

Further, the surface of the proximal end of the channel joint portion 22 has a gear groove 22c. This gear groove 22c is engaged with a sending gear 56 that is rotated by a motor (not illustrated). The motor and sending gear 56 comprise the above-mentioned drive mechanism 26. Now, the position of the channel joint portion 22 illustrated in Fig. 8, in other words, the position before the channel joint portion 22 is linked with the first insertion portion 1a of the insertion portion 1, is set as the retreated position of the endoscope washer device 40.

Further, the control circuit 200 is electrically connected to a sensor 57 illustrated in Fig. 8. This sensor 57 supplies a detection signal to the control circuit 200 when one of the two convex portions 22b of the proximal end of the channel joint portion 22 passes by. In other words, these two convex portions 22b are provided on the proximal-end portion of the channel joint portion 22 in the movement direction of the channel joint portion 22 at a distance that matches the predetermined advancing distance or the retreating distance of the channel joint portion 22. Then upon two convex portions 22b passing by the sensor 57, the motor of the drive mechanism 26 is supplied with a stopping signal from the control circuit 200, so as to stop. Therefore, the predetermined movement distance of the channel joint portion 22 for advancing towards or retreating from the insertion portion 1 is controlled.

Three fluid sending tubes 58 pass through the inside of the channel joint portion 22, and these fluid sending tubes 58 extend from the proximal end of the channel joint portions 22. Further, the channel joint portion 22 passes through a hole in the side wall of the cleaning sink 2 wherein a sealing member 55 in a ring shape formed of an elastic material is provided.

This sealing member 55 adheres to the periphery face of the channel joint portion 22 by the elastic form thereof, and accordingly the inside and the outside of the cleaning sink 2 can retain water-tightness, even if the channel joint portion 22 moves in the direction of being approximately perpendicular to the wall face of the cleaning sink 2.

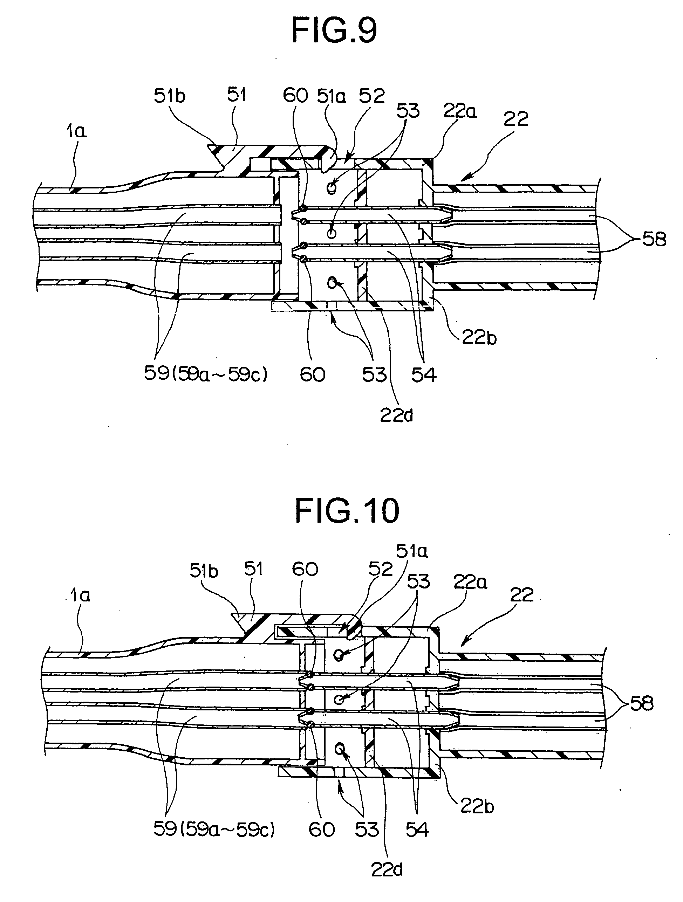

Next, the linking portion 22a of the channel joint portion 22 and the first insertion portion 1a of the endoscope 1A will be described in detail while referencing Fig. 9. Fig. 9 is a cross-sectional diagram of the linking portion 22a between the channel joint portion 22 and the first insertion portion 1a of the insertion portion 1.

As illustrated in Fig. 9, the three endoscope channels 59, i.e., a treatment equipment channel 59a, an air and water sending channel 59b, and a suction channel 59c, wherein both ends are open from the first insertion portion 1a that is in an approximately cylindrical shape as described above, to the tip portion of the second insertion portion 1b pass through the inside of the insertion portion 1.

The linking portion 22a of the channel joint portion 22 has an approximately cylindrical shape wherein one end is closed by the end face member 22e. This linking portion 22a has a similar internal shape as the external shape of the first insertion portion 1a, and has a slightly larger inner diameter than the external diameter of the first insertion portion 1a.

The linking portion 22a has a hook hole 52 that is a slot for hooking the clip section of the hook 51 on the first insertion portion 1a, and multiple small holes 53. The length of the hook hole 52 in the lengthwise direction is only the predetermined amount longer than that of the clip section to the lengthwise direction of the hook 51.

Further, the linking portion 22a has a plate member 22d for closing the channel further on the proximal end from the positions of the hook hole 52 and the small holes 53. The inside of this linking portion 22a has three channel cleaning nozzles 54 that are circulation fluid supply ducts that pass through the plate member 22d.

These channel cleaning nozzles 54 also pass through the end face member 22e of the linking portion 22a, and are supported at two points and fixed by the plate member 22d and the end face member 22e. Further, the channel cleaning nozzle 54 is a tube wherein both ends have an approximately conical shape, and the proximal ends thereof are connected with the fluid sending tube 58 that pass through the inside of the channel of the channel joint portion 22. Further, an O-ring 60 is provided on the tip portion of the channel cleaning nozzle 54.

Now, at the position of the channel joint portion 22 illustrated in Fig. 9, the position of the channel cleaning nozzle 54 which is a fluid supply channel serves as the second retreating position of the endoscope washer device 40.

Next, the operation during use of the endoscope washer device 40 relating to the present embodiment will be described in detail.

First, as illustrated in Fig. 2, a worker opens the top cover 3 of the endoscope washer device 40, and sets the insertion portion 1 of the endoscope 1A that is to be cleaned and sterilized in the cleaning sink 2 of the endoscope washer device 40. Now, in the case that the various endoscope channels 69 that pass through, or run along the outside of, the universal cord 1c and the operating unit 37 wherein the insertion portion 1 is removed, are a discardable type, they are pulled out or removed from the operating unit 37 and the universal cord 1c, and are discarded as predetermined. Further, the operating unit 37 from which the various endoscope channels 69 have been removed and the universal cord 1c are cleaned and sterilized once or twice daily, according to the degree of contamination.

In this case, the worker sets the first insertion portion 1a of the insertion portion 1 in the first holding member 4 of the cleaning sink 2 so that the proximal-end face of the first insertion portion 1a faces the tip face of the channel joint portion 22. Further, the worker sets the first insertion portion 1a into the first holding member 4 so that the hook 51 of the first insertion portion 1a and the hook hole 52 of the linking portion 22a of the channel joint portion 22 are on a straight line in the lengthwise direction of the channel joint portion 22.

Further, the worker sets the second insertion portion 1b of the insertion portion 1 while arbitrarily selecting from multiple second holding members 5. In the situation of setting the second insertion portion 1b into the second holding member 5, the worker should set as if drawing a smooth curved line with the bending portion of the second insertion portion 1b

The detecting sensor of the first holding member 4, here an optical sensor, recognizes and detects that the first insertion portion 1a of the insertion portion 1 is set in the predetermined direction, and supplies a detection signal to the control circuit 200. The control circuit 200, upon receives this detection signal, supplies a drive signal to the drive mechanism 26, and the motor inside the drive mechanism 26 rotates the sending gear 56 (see Fig. 8).

The rotation direction of this sending gear 56 is the direction of the arrow A illustrated in Fig. 8. Therefore, since the gear groove 56a of the sending gear 56 and the gear grove 22c of the channel joint portion are engaged, when the sending gear 56 rotates in the direction of the arrow A, the channel joint portion 22 automatically advances in the direction of the arrow B, in other words, toward the first insertion portion 1a of the insertion portion 1.

As illustrated in Fig. 9, in the situation that the channel joint portion 22 is pressed into the proximal-end portion of the first insertion portion 1a of the insertion portion 1, the chamfered face of the clip section 51a of the hook 51 makes contact with the tip portion of the linking portion 22a, and the clip section 51a of the hook 51 is pushed upwards in Fig. 9. Further, by the channel joint portion 22 advancing toward the insertion portion 1, the clip section 51a of the hook 51 is pressed into the hook hole of the linking portion 22a. As illustrated in Fig. 10, the tip of the channel cleaning nozzle 54 is advanced toward the insertion portion 1 to the position wherein it is inserted in the three endoscope channels 59 that pass through the insertion portion 1.

With the position of the channel joint portion 22 illustrated in Fig. 10, the convex portion 22b is positioned and fixed on the proximal-end surface of the channel joint portion 22 so that the sensor 57 can detect the convex portion 22b of the channel joint portion 22. In other words, the channel joint portion 22, under the control of the control circuit 200, is advanced toward the insertion portion 1 up to the position in Fig. 10, and to the position wherein the tip of the linking portion 22a makes contacts with the end face of the first insertion portion 1a and stops.

Further, as illustrated in Fig. 10, by the O-ring 60 that is provided on the tip portion of the channel cleaning nozzle 54 making contact with the open portion of the endoscope channel 59, the channel cleaning nozzle 54 and the endoscope channel 59 communicate while retaining water-tightness.

Further, the worker injects a predetermined amount of liquid detergent into the cleaning sink 2 so as not to directly come into contact with the insertion portion 1.

Next, the worker closes the top cover 3, presses the start switch that is on the operating panel 41 of the endoscope washer device 40 as illustrated in Fig. 2, and begins the cleaning process of the endoscope washer device 40.

Following is a detailed description of the cleaning process of the endoscope washer device 40.

The endoscope washer device 40 begins the cleaning process of the insertion portion 1 of the endoscope 1A when the start switch on the operating panel 41 is pressed. First, the water supply valve 17 of the endoscope washer device 40 opens, and a dilution fluid is supplied from the spigot 16 that is the water supply source, in this case tap water is supplied inside the sink of the cleaning sink 2 from the nozzle 18, having passed through the water supply filter 10 that is on the water supply channel 15 and the check valve 11.

The water supply continues until the water level of the tap water makes contact with one of the liquid face detecting units of the water level detecting sensor 6 on the wall face of the cleaning sink 2. When the tap water makes contact with the first liquid face detecting unit of the water level detecting sensor 6, a detection signal is supplied from the water level detecting sensor 6 to the control circuit 200. The control circuit 200 to which the detection signal is supplied supplies a drive signal to the electromagnetic opening/closing valve 25 and the channel interior cleaning pump 21, opens the valve inside the electromagnetic opening/closing valve 25, and drives the channel interior cleaning pump 21. At this time, the tap water continues to be supplied to the inside of the cleaning sink 2 from the nozzle 18.

Accordingly, the cleaning fluid, wherein the detergent and the tap water are mixed, stored in the cleaning sink 2, passes through the channel interior cleaning channel 19 that communicates to the circulating fluid cleaning/sterilizing channel 9 from the circulating fluid suction opening 20, and is suctioned by the channel interior cleaning pump 21.

The channel interior cleaning pump 21 sends the suctioned cleaning fluid towards the check valve 36 of the channel interior cleaning channel 19. The sent cleaning fluid passes through the channel interior cleaning channel 19, passes by the check valve, and is sent to the branch channel 27 that communicates with the channel interior cleaning channel 19, and arrives at the electromagnetic opening/closing valve 25. Here, the cleaning fluid in the branch channel 27 cannot flow into the air supply channel 30 because of the check valve 29 on the air supply channel 30.

Since the valve of the electromagnetic opening/closing valve 25 is open, the cleaning fluid is divided into three channels by the branch channel 27. Then the cleaning fluid passes through the electromagnetic opening/closing valves 24a through 24c and the fluid sending tubes 58 of the channel joint portion 22, and from the channel cleaning nozzle 54, is supplied, or in other words sprayed, into the endoscope channel 59 of the operating unit 37 of the insertion portion 1 at a predetermined flow rate.

While the above-described endoscope washer device 40 continues operation, the water level sensor 6 supplies a detection signal to the control circuit 200 at the point that the liquid face of the cleaning fluid supplied to the cleaning sink 2 makes contact with the second liquid face detecting unit of the water detection sensor 6. The control circuit 200 that is supplied with this detection signal supplies a drive signal to the water supply valve 17 and closes the water supply valve 17.

Therefore, the spigot 16 stops supplying tap water from the nozzle 18 into the cleaning sink 2 due to the valve inside the water supply valve 17 closing. Further; the second liquid face detecting unit of the water detection sensor 6 is set at a height wherein the insertion portion 1 is completely submerged under water from the floor face of the cleaning sink 2. In other words, the insertion portion 1 that is set in the cleaning sink 2 is sufficiently covered by the cleaning fluid.

Next, a drive signal is supplied to the circulating fluid cleaning/sterilizing pump 8 by the control circuit 200, and the circulating fluid cleaning/sterilizing pump 8 begins driving. In this instance, the channel interior cleaning pump 21 continues to drive. Then the cleaning fluid inside the cleaning sink 2 passes through two paths from the circulating fluid suction opening 20 and is discharged from of the cleaning sink 2. In other words, the two paths are the above-described first circulating fluid path and the second circulating fluid path.

The cleaning fluid that circulates through the first circulating fluid path performs cleaning primarily of the end face of the first insertion portion 1a of the insertion portion 1, and the endoscope channels 59 that pass through the inside of the insertion portion 1. Further, the circulating fluid discharge opening 7 performs cleaning of the external face of the insertion portion 1 with the cleaning fluid that circulates through the second circulating fluid path.

The cleaning process of the endoscope washer device 40 is performed for a predetermined duration of time, and during this cleaning process, the channel joint portion 22 makes at least one advancing movement and retreating movement with regard to the insertion portion 1. Specifically, the channel joint portion 22 makes at least one reciprocal movement between the position illustrated in Fig. 10 wherein the tip portion of the channel cleaning nozzle 54 of the linking portion 22a is inserted into the endoscope channels 59 of the first insertion portion 1a, and the position illustrated in Fig. 9 wherein the channel cleaning nozzle 54 of the linking portion 22a is separated from the endoscope channels 59, during the cleaning process of the endoscope washer device 40.

By the control circuit 200 supplying a drive signal to the motor of the drive mechanism 26 a predetermined length of time, at least once, the sending gear 56 illustrated in Fig. 8 rotates in the direction of the arrow A and the opposite direction of the arrow A. Therefore, the channel joint portion 22 is moved in the two directions, to advance toward the insertion portion 1 and to retreat.

The movement distance of these two directions is approximately 2 mm for example, and the spacing of the two convex portions of the proximal end of the channel joint portion 22 is also positioned with same 2 mm spacing to match the movement distance thereof. The movement of the two direction of the channel joint portion 22 is performed during the execution of the cleaning process of the insertion portion 1 of the endoscope 1A.

Now, so that the channel joint portion 22 can move sufficiently in the two directions, the hole length in the lengthwise direction of the hook hole 52 of the linking portion 22a has a length of the clip section 51a in the lengthwise direction of the hook 51 of the insertion portion 1 to be fit in, in other words, a length that is the length of the portion to be fit in plus at least 2 mm.

Further, at the position of the channel joint portion 22 illustrated in Fig. 9, in other words, at the state wherein the endoscope channels 59 (59a through 59c) of the insertion portion 1 and the tip portion of the linking portion 22a of the channel joint portion 22 is separated, the flow of the cleaning fluid that is discharged to the end face of the first insertion portion 1a of the insertion portion 1 is separated into a flow that passes through the endoscope channels 59 (59a through 59c) and flows out from the tip of the second insertion portion 1b to the cleaning sink 2, and the flow that flows out from the small holes 53 of the linking portion 22a into the cleaning sink 2.

At the position of the channel joint portion 22 illustrated in Fig. 10, due to the O-ring 60 of the channel cleaning nozzle 54 of the linking portion 22a making direct contact with the opening edge of the endoscope channels 59 (59a through 59c) of the insertion portion 1, the flow of the cleaning fluid passes through the endoscope channels 59 of the insertion portion 1, and flows out from the tip of the second insertion portion 1b to the cleaning sink 2.

Therefore, cleaning fluid that circulates through the first path primarily performs cleaning on the endoscope channels 59 (59a through 59c) that pass through the inside of the insertion portion 1, and performs cleaning on the end face of the first insertion portion 1a of the insertion portion 1, by the movement in two directions of advancing toward or retreating from the insertion portion 1 of the channel joint portion 22.

Further, during the predetermined movement of the endoscope washer device 40, the flow rate sensor of the electromagnetic opening/closing valves 24a through 24c constantly measure the flow rate of the cleaning fluid that passes through the inside of the electromagnetic opening/closing valves 24a through 24c. In the case that cleaning fluid passing through the inside of the electromagnetic opening/closing valves 24a through 24c is below the predetermined flow rate, the flow rate sensor supplies a warning signal to the control circuit 200, and the control circuit 200 displays a warning on the operating panel 41 and supplies an instruction signal to sound a warning sound to a buzzer (not illustrated).

When the warning signal is supplied to the control circuit 200 for a predetermined length of time, the control circuit 200 supplies a stop signal to the various equipment of the endoscope washer device 40, and stops each of the components being driven.

When the predetermined length of cleaning process time has passed, the control circuit 200 supplies a stopping signal to the driving circulating fluid cleaning/sterilizing pump 8 and the channel interior cleaning pump 21. Then the circulating fluid cleaning/sterilizing pump 8 and the channel interior cleaning pump 21 stop driving.

Further, the control circuit 200 supplies a drive signal to the switching valve 13 and the draining pump 28. The switching valve 13 wherein a drive signal is supplied opens the valve therein to the side of the draining channel 35, and communicates the cleaning sink 2 and the draining channel 35.

Simultaneous with the operation of this switching valve 13, the draining pump 28 begins driving by the drive signal from the control circuit 200, and sends the cleaning fluid inside the cleaning sink 2 from the draining channel 35 of the cleaning sink 2 side to the draining hose 42.

Therefore, the cleaning fluid that is stored within the cleaning sink 2 is discharged from the endoscope washer device 40. Then, due to the cleaning fluid having been sufficiently discharged, after the draining pump 28 has operated for a predetermined length of time, the control circuit 200 supplies a stop signal to the draining pump 28, and supplies a drive signal to the switching valve 13.

Thus, the draining pump 28 stops driving, and the switching valve 13 closes the inner valve.

After the cleaning process of the endoscope washer device 40 described above is completed, a rinsing process for rinsing the insertion portion 1 starts.

The rinsing process of the endoscope washer device 40 will be described below, however the operations similar to the cleaning process will be described in an abbreviated manner.

First, the drive signal of the control circuit 200 is supplied to the water supply valve 17, the water supply valve 17 opens the valve therein, and tap water from the spigot 16 is supplied into the sink of the cleaning sink 2 from the nozzle 18. As with the cleaning process, when the tap water makes contact with the first liquid detecting unit of the water level detecting sensor 6, the valve inside the electromagnetic opening/closing valve 25 opens, and the channel interior cleaning pump 21 begins to drive. At this time, the tap water continues to be supplied from the nozzle 18 inside the cleaning sink 2.

Since the valve of the electromagnetic opening/closing valve 25 is open, the rinse water that is tap water is divided into three channels by the branch channel 27, and a predetermined flow rate is sprayed from the channel cleaning nozzle 54 onto the side face of the first insertion portion 1a of the insertion portion 1.

While the endoscope washer device 40 continues the above-described operation, when the liquid face of the tap water makes contact with the second liquid face detecting unit of the water level sensor 6, the water supply from the nozzle 18 into the cleaning sink 2 is stopped. At this time, the insertion portion 1 that is set inside the cleaning sink 2 is sufficiently submerged under water.

Next, the circulating fluid cleaning/sterilizing pump 8 begins to drive, and the channel interior cleaning pump 21 also continues to drive, and the rinse water inside the cleaning sink 2 circulates from the circulating fluid suction opening 20 through the first circulating fluid path and the second circulating fluid path.

The rinse water that circulates through the first circulating path primarily rinses the side face of the first insertion portion 1a of the insertion portion 1 and the various channels that pass through the inside of the insertion portion 1, and the rinse water that circulates through the second circulating path rinses the exterior face of the insertion portion 1 by the circulating fluid discharge opening 7.

The rinsing process of the endoscope washer device 40 is performed for a predetermined length of time, and during the execution of this rinsing process, the channel joint portion 22 makes at least one advancing movement and retreating movement regarding the insertion portion 1.

At the position of the channel joint portion 22 illustrated in Fig. 9, in other words, at the state wherein the endoscope channels 59 (59a through 59c) of the insertion portion 1 and the tip portion of the linking portion 22a of the channel joint portion 22 is separated, the flow of the rinse water that is discharged to the end face of the first insertion portion 1a is separated into a flow that passes through the endoscope channels 59 (59a through 59c) and flows out from the tip of the second insertion portion 1b, and the flow that flows out from the small holes 53 of the linking portion 22a into the cleaning sink 2.

At the position of the channel joint portion 22 illustrated in Fig. 10, due to the O-ring 60 of the channel cleaning nozzle 54 of the linking portion 22a making direct contact with the opening edge of the endoscope channels 59 (59a through 59c) of the insertion portion 1, the flow of the rinse water passes through the endoscope channels 59 (59a through 59c) of the insertion portion 1, and flows out from the tip of the second insertion portion 1b to the cleaning sink 2.

Therefore, rinse water that circulates through the first path primarily rinses the endoscope channels 59 (59a through 59c) that pass through the inside of the insertion portion 1, and rinses the end face of the first insertion portion 1a of the insertion portion 1, by the movement in two directions of advancing toward or retreating from the insertion portion 1 of the channel joint portion 22.

Further, while the rinse water is circulating through the first circulating fluid path, the flow rate sensor of the electromagnetic opening/closing valves 24a through 24c constantly measure the flow rate of the rinse water that passes through the inside of the electromagnetic opening/closing valves 24a through 24c. In the case that the rinse water passing through the inside of the electromagnetic opening/closing valves 24a through 24c is below the predetermined flow rate, the control circuit 200 sends instructions to the operating panel 41 to display a warning and to the buzzer to sound a warning sound. When the warning signal is continued for a predetermined length of time, the various equipment of the endoscope washer device 40 stops operating.

When the predetermined length of rinsing process time has passed, the circulating fluid cleaning/sterilizing pump 8 and the channel interior cleaning pump 21 stop driving. Further, the switching valve 13 opens the valve therein to the side of the draining channel 35, and communicates the cleaning sink 2 and the draining channel 35, and the draining pump 28 begins to drive.

Therefore, the rinse water that is stored in the cleaning sink 2 is discharged from the endoscope washer device 40. In order for the rinse water to be sufficiently discharged, the draining pump 28 drives for a predetermined amount of time, and then stops.

At this time, the channel joint portion 22 stops at the position that is advanced toward the first insertion portion 1a of the insertion portion 1, in other words, the position illustrated in Fig. 10 wherein the tip portion of the channel cleaning nozzle 54 of the linking portion 22a is inserted into the endoscope channels 59 (59a through 59c) of the insertion portion 1.

Next, the control circuit 200 supplies a drive signal to the compressor 31. The compressor 31 begins to drive, and supplies compressed air to the air supply channel 30. This compressed air passes through the check valve 29 in the air supply channel 30, blows into the branch channel 27, passes through the fluid sending tube 58 of the channel joint portion 22 and the channel cleaning nozzle 54, and is blown into the endoscope channels 59 (59a through 59c) of the insertion portion 1.

Further, the compressed air from the compressor 31 cannot be blown into the channel of the channel interior cleaning channel 19 because of the check valve 36.

Further, the compressed air that is blown into the endoscope channels 59 (59a through 59c) sends the remaining water inside the endoscope channels 59 (59a through 59c) toward the tip of the second insertion portion 1b of the insertion portion 1, and is blown out from the opening in the tip of the endoscope channels 59 (59a through 59c). Therefore, the remaining water inside the endoscope channels 59 flows out into the sink of the cleaning sink 2 from the opening in the tip of the endoscope channels 59 (59a through 59c), and the inside of the endoscope channels 59 (59a through 59c) is dried.

After the above described rinsing process is completed, the endoscope washer device 40 follows by performing a sterilization process. The sterilizing process of the endoscope washer device 40 will be described below, however the operations similar to the cleaning process and rinsing process will be described in an abbreviated manner. Further, until the above-described rinsing process is completed, the switching valve 13 closes off the chemical collecting channel 23 and the draining opening 14 by the inner valve.

Next, the sterilization process of the endoscope washer device 40 will be described in detail.

First, the control circuit 200 supplies a drive signal to the injecting pump 34, and the sterilizing fluid that is stored within the tank of the sterilizing fluid tank is sent into the side of the sterilizing fluid injection channel 33 connected to the water supply channel 15.

This sterilizing fluid passes through the check valve 12 in the sterilizing fluid injection channel 33, is sent into the water supply channel 15, and flows into the sink of the cleaning sink 2 from the nozzle 18. At this time, due to the backflow valve 11 of the water supply channel 15, the sterilizing fluid does not flow to the side of the water filter 10 of the water supply channel, and is all sent to the side of the nozzle 18.

When the insertion portion 1 that is set in the cleaning sink 2 is sufficiently submerged in the sterilizing fluid, the control circuit 200 supplies a stop signal to the injecting pump 34, and supplies a drive signal to the channel interior cleaning pump 21. Specifically, the timing of supplying the stop signal to the injecting pump 34 and the drive signal to the channel interior cleaning pump 21 by the control circuit 200 is the time at which the second liquid face detecting unit of the water level detecting sensor 6 detects the liquid face of the sterilizing fluid.

The channel interior cleaning pump 21 receives a drive signal from the control circuit 200 and begins to drive. Thus, as with the cleaning process, the sterilizing fluid circulates throughout the first circulating fluid path.

Therefore, the sterilizing fluid is discharged into the channels of the endoscope channels 59 (59a through 59c) of the insertion portion 1 by the channel cleaning nozzle 54 of the channel joint portion 22. Further, during the execution of the sterilizing process, the channel joint portion 22 is supplied a drive signal at least once from the control circuit 200 to the drive mechanism 26, and performs at least one reciprocating advancing movement and retreating movement toward and away from the insertion portion 1. At the time of retreating, the sterilizing fluid is discharged onto the proximal-end face of the first insertion portion 1a of the insertion portion 1.

On the other hand, at the position of the channel joint portion 22 illustrated in Fig. 9, in other words, at the state wherein the endoscope channels 59 (59a through 59c) of the insertion portion 1 and the tip portion of the linking portion 22a of the channel joint portion 22 is separated, the flow of the sterilizing fluid that is discharged to the end face of the first insertion portion 1a of the insertion portion 1 is separated into a flow that passes through the endoscope channels 59 (59a through 59c) and flows out from the tip of the second insertion portion 1b, and the flow that flows out from the small holes 53 of the linking portion 22a into the cleaning sink 2.

Further, during the sterilizing process of the endoscope washer device 40, a drive signal can be supplied from the control circuit 200 to the circulating fluid cleaning/sterilizing pump 8, and the circulating fluid cleaning/sterilizing pump 8 can be driven so that the sterilizing fluid is circulated through the circulating paths.

Therefore, the external surface of the insertion portion 1 is exposed to the sterilizing fluid that is stored inside the sink of the cleaning sink 2, and the first insertion portion 1a and the endoscope channels 29 (59a through 59c) therein are sterilized by the sterilizing fluid that circulate through the first circulating path.

After a predetermined length of time has passed, the control circuit 200 supplies a drive signal to the switching valve 13. The switching valve 13 that receives the drive signal opens the inner valve so as to communicate the chemical collecting channel 23 with the drain opening 14. In other words, the chemical collecting channel 23 communicates with the inside of the sink of the cleaning sink 2. Therefore, the sterilizing fluid that is stored in the cleaning sink 2 is collected in the sterilizing fluid tank 32.

Thus, the endoscope washer device 40 completes the sterilizing process, and begins again the rinsing process described above. This rinsing process of the endoscope washer device 40 is the same as the above described operation, and therefore description thereof will be omitted.

Further, as described above, in the rinsing process, while the compressed air from the compressor 31 is removing water from the endoscope channels 59 (59a through 59c) that pass through the inside of the insertion portion 1 for a predetermined length of time, the draining pump 28 drives, and the rinse water inside the sink of the cleaning sink 2 is discharged outside of the endoscope washer device 40 from the draining hose 42.

With the rinsing process after the sterilization process of the endoscope washer device 40, the draining pump 28 continues driving for a predetermined length of time after the compressor 31 receives a stop signal from the control circuit 200 and stops driving. During the drive of the draining pump 28, the switching valve 13 is open so that the inner valve enables the draining channel 35 and the drain opening 14 to communicate. As a result, approximately all of the rinse water inside the cleaning sink 2 is discharged from the endoscope washer device 40 from the drain opening 14.

Lastly, the various electromagnetic valves and so forth that are controlled by the control circuit 200 return to the predetermined beginning set positions, and the endoscope washer device 40 completes the cleaning and sterilization of the insertion portion 1.

After this, the worker opens the top cover 3 of the endoscope washer device 40, and removes the first insertion portion 1a of the insertion portion 1 from the linking portion 22a of the channel joint portion 22.

Specifically, the end portion of the hook 51 of the first insertion portion 1a of the insertion portion 1, in other words the clip section of the hook 51 and the pinching section 51b on the other side, is pushed with the thumb or the like, and the clip section 51a of the hook 51 that is hooked onto the hook hole 52 of the channel joint portion 22 is released, and the first insertion portion 1a and the channel joint portion 22 are separated.

Further, more specifically, when the pinching section 51b of the hook 51 is pressed in the downward direction in Fig. 9, the clip section 51a of the hook 51 of the first insertion portion 1a moves in the upward direction in Fig. 9, and separates from the hook hole 52 of the linking portion 22a of the channel joint portion 22.

Then the worker separates the first insertion portion 1a of the insertion portion 1 from the first holding member 4, and separates the second insertion portion 1b from the second holding members 5, and completes the work by removing the insertion portion 1 from the cleaning sink 2 of the endoscope washer device 40.

As a result of the above, the endoscope washer device 40 of the endoscope washer system 300 relating to the first embodiment automatically inserts the tips of the channel cleaning nozzle 54 of the linking portion 22 of the channel joint portion 22 into the multiple endoscope channels 59 (59a through 59c) that pass through the inside of the insertion portion 1, and the cleaning fluid, the rinse water, or the sterilizing fluid is sprayed out from the channel cleaning nozzle 54. Further, the channel joint portion 22 makes at least one advancing movement or retreating movement toward the insertion portion 1, and therefore the cleaning fluid, the rinse water, or the sterilizing fluid is sprayed onto the end face of the first insertion portion 1a of the insertion portion 1 from the channel cleaning nozzle 54.

Therefore, the worker can easily set the insertion portion 1 into the cleaning sink 2 without making direct contact with the multiple cleaning tubes such as the endoscope channels 59 (59a through 59c).

Further, in the case that a lower flow rate than that predetermined of the cleaning fluid, rinse water, or sterilizing fluid that circulates through the inside of the channel joint portion 22 is passed through the branch channel 27, the control device 200 generates a warning message to the operating panel and generates an internal warning sound, by the flow rate gauge that is provided on the electromagnetic opening/closing valves 24a through 24c of the branch channel 27, and alerts the worker to an abnormality. When this warning is continued a predetermined length of time, the endoscope washer device 40 automatically stops operating the various equipment.

Therefore, the first insertion portion 1a of the insertion portion 1 of the endoscope 1A, and the endoscope channels 59 (59a through 59c) that pass through the inside thereof, are certain to be subjected to cleaning and sterilization.

Further, the endoscope 1A of the present embodiment is arranged such that the insertion portion 1 is removed from the operating unit 37 and the insertion portion 1 can be easily set into the cleaning sink 2 of the endoscope washer device 40.

Therefore, according to the endoscope washer system 300 of the present embodiment, time needed to connect the cleaning tubes can be eliminated, and cases wherein the worker forgets to connect the cleaning tubes are eliminated, while the need to check whether or not these are correctly connected is also unnecessary, and the efficiency of the endoscope is improved.

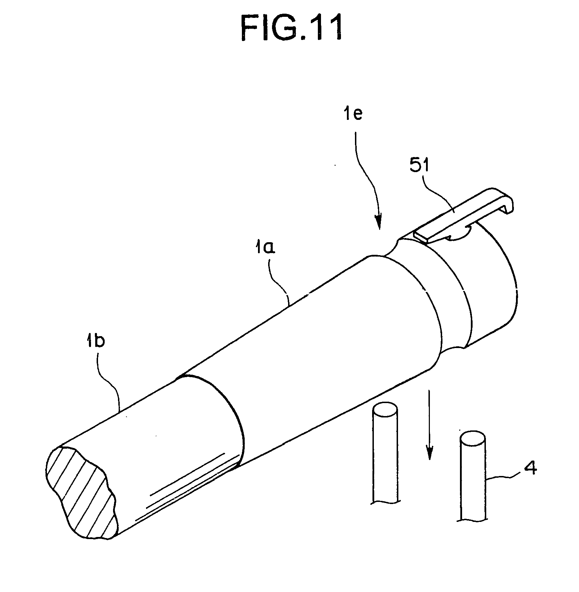

Now, as illustrated in Fig. 11, the first insertion portion 1a is approximately perpendicular to the axis of the lengthwise direction of the periphery, and is a groove for positioning wherein the first holding member 4 of the endoscope washer device 40 holds the first insertion portion 1a, and a guide groove 1e can be formed that is a guide portion. Further, as illustrated in Fig. 12, a guide groove 1e' can be formed only on the side portion of the first insertion portion 1a.

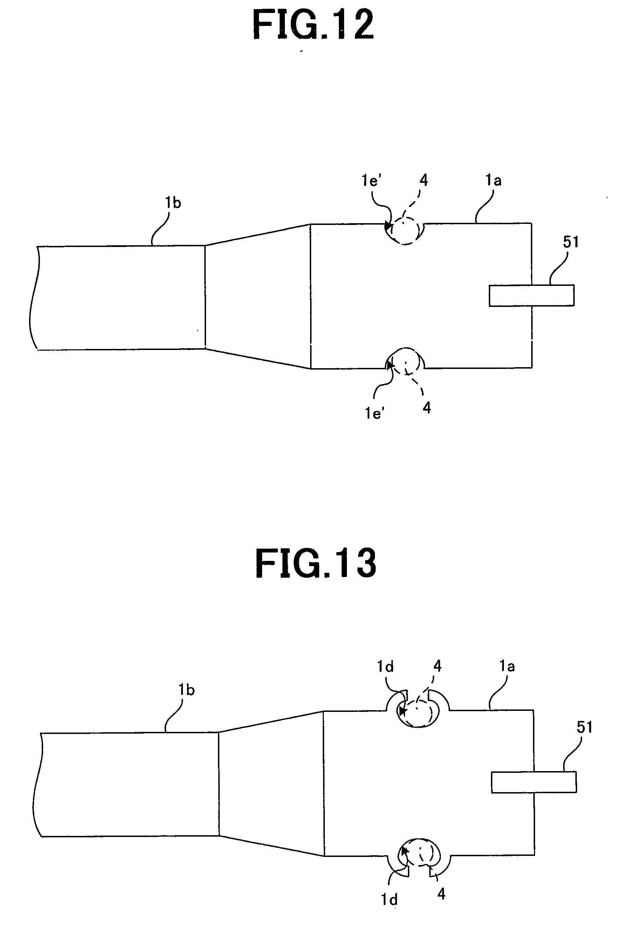

Further, as illustrated in Fig. 13, a guide hole 1d that is a guide portion wherein the first holding member 4 of the endoscope washer device 40 passes through and holds, can be provided on the side portion of the first insertion portion 1a.

As a result of the above, due to the guide grooves 1e and 1e' and the guide hole 1d illustrated in Fig. 11 through Fig. 13 being provided, the first insertion portion 1a to be held by the first holding member 4 of the endoscope washer device 40 is positioned in a sure manner. Therefore, the insertion portion 1 is set in the predetermined position within the cleaning sink 2 of the endoscope washer device 40 in a sure manner.

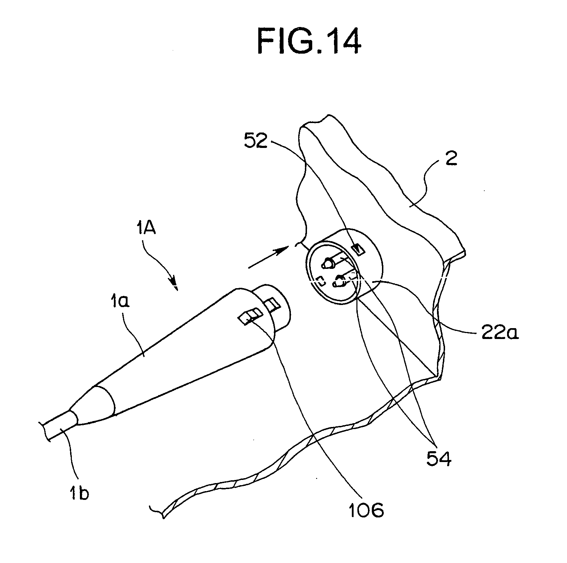

Further, as illustrated in Fig. 14, the linking portion 22a of the channel joint portion 22 can be fixed to one wall face of the cleaning sink 2. Therefore, the insertion portion 1 of the endoscope 1A can be configured so that a detachable hook 106 of the first insertion portion 1a is fit into the hook hole 52 of the linking portion 22a by the worker, and the first insertion portion 1a is linked.

At the time of linking the linking portion 22a of the channel joint portion 22 and the first insertion portion 1a of the insertion portion 1, the circulating fluid such as the cleaning fluid can easily flow into the inside of the cleaning sink 2 by providing a small amount of play. Specifically, the circulating fluid from the channel cleaning nozzle 54 is sprayed out toward the endoscope channels 59 (59a through 59c) on the side face of the first insertion portion 1a of the insertion portion 1. This circulating fluid is divided into the circulating fluid that passes through the inside of the endoscope channels 59 (59a through 59c) and flows into the sink of the cleaning sink 2 from the tip of the insertion unit 1b, and the circulation fluid that collides with the end face of the first insertion portion 1a and flows into the sink of the cleaning sink 2 from the small holes 53 of linking portion 22a.

Further, the opening portion of the endoscope channels 59 (59a through 59c) of the first insertion portion 1a and the channel cleaning nozzle 54 separates somewhat due to the above described play, by the spraying force of the circulating fluid.

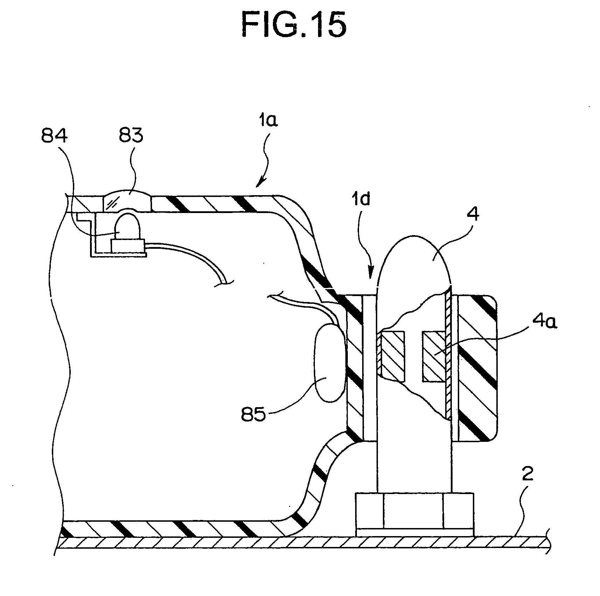

Further, as illustrated in Fig. 15, the first insertion portion 1a can house a light-emitting diode (LED) 84 serving as notifying means, and an irradiation window 83 for irradiating the light of this LED 84, and a lead switch 85 that reacts to a magnet 4a that is positioned near the guide hole 1d and is housed in the first holding member 4 of the endoscope washer device 40.

Therefore, in the case that the first insertion portion 1a is correctly positioned in the cleaning sink 2 of the endoscope washer device 40, the lead switch 85 reacts to the magnet 4a of the first holding member 4 and turns ON, and the LED 84 illuminates. Thus, the worker can easily confirm that the first insertion portion 1a is set in the correct position of the cleaning sink 2.

Further, the first insertion portion 1a can provide an notifying means such as a buzzer along with the illumination of the LED 84, or in place of the LED 84.

### [Second Embodiment]

Next, an endoscope washer device 40' of the endoscope washer system 300 relating to a second embodiment will be described. Now, the description of the endoscope washer device 40' of the present embodiment will use the same reference numerals (with a "'" added to the reference numerals of the first embodiment) for components wherein the configuration is similar to that of the endoscope washer device 40 of the first embodiment, and description thereof will be omitted. Also, operation and advantages thereof will also be described in an abbreviated manner.

Further, the endoscope 1A' that is used in the present embodiment has the form of the generally used operating unit 37'.

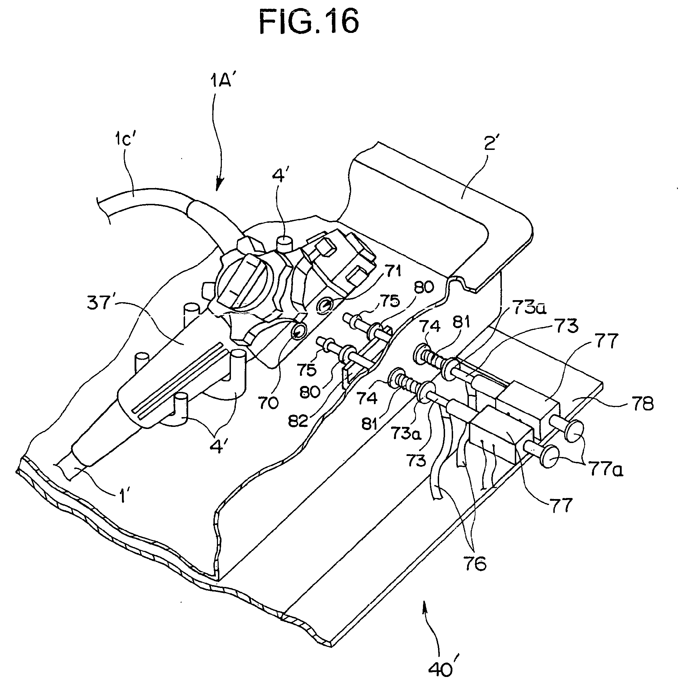

Fig. 16 is a partial configuration diagram illustrating an internal portion wherein the endoscope 1A' is set in the sink of the cleaning sink 2 of the endoscope washer device 40' relating to the present embodiment.

The endoscope 1A' illustrated in Fig. 16 has an operating unit 37' that has an operating knob and various switches and the like, and an insertion portion 1' that extends from the operating unit 37' and a universal cord 1c'. Further, one side face of the operating unit 37' has various opening portions such as an air and water sending channel 70 that is an endoscope channel that communicates to the inside of the insertion portion 1', and an suction channel 71.

This endoscope 1A' is set in a predetermined position within the cleaning sink 2' by the multiple first holding members 4' and the multiple second holding members 5' provided within the sink of the cleaning sink 2' of the endoscope washer device 40'. The placement of the operating unit 37' of the endoscope 1A' into the first holding members 4' will be described in detail. The operating unit 37' is held and fixed in the predetermined position by three first holding members 4' of the endoscope washer device 40' so that the opening portions on one side face of the air and water sending channel 70 and the suction channel 71 are facing toward the later-described two channel cleaning nozzles 73.

The first holding members 4' are in the position so that the openings of the air and water sending channel 70 and the suction channel 71 facing each of the tips of the two channel cleaning nozzles 73, and are positioned on the floor face of the cleaning sink 2' so that the center of these openings each are positioned approximately on the lengthwise axis of the two channel cleaning nozzles 73.

To describe further, two of the three first holding members 4' are in a U-shape on the side wherein the insertion portion 1' is extended according to the gripping portion form of the operating unit 37', and the predetermined gripping portion of the operating unit 37' is narrowed. Further, the remaining one first holding member 4' is positioned so as to make direct contact with the side face opposite from the side face of the operating unit 37' that has the air and water sending channel 70 that is near the universal cord 1c' extending from the operating unit 37' and the suction channel 71, and is fixed onto the floor face of the cleaning sink 2'.

Next, the configuration of the above-described channel cleaning nozzle 73 will be described in detail. Two channel cleaning nozzles 73 pass through the side wall of the cleaning sink 2' of the endoscope washer device 40'. A sealing member 74 to retain water-tightness is provided on this side wall portion of the cleaning sink 2' wherein the channel cleaning nozzles 73 are passing through.

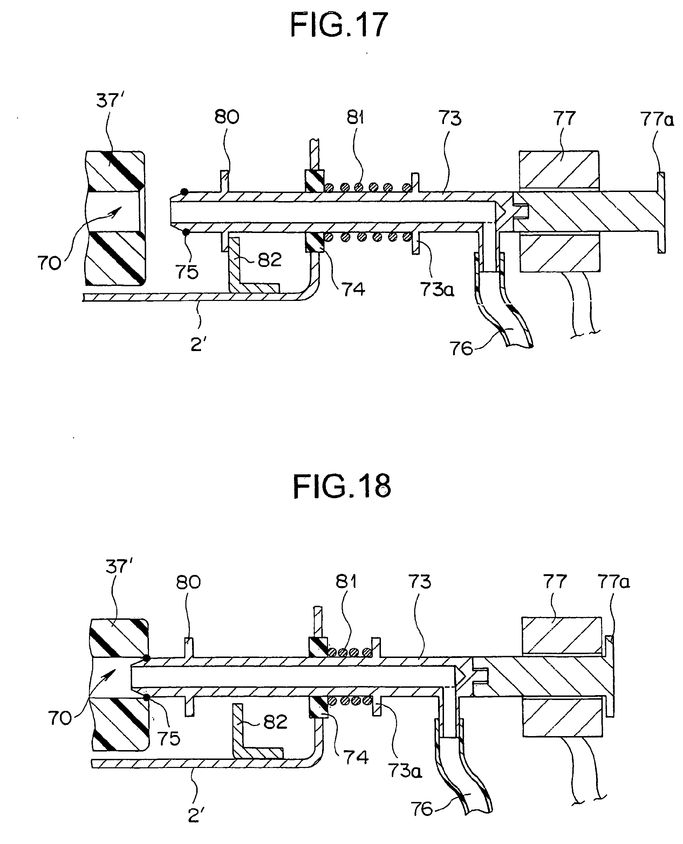

Since these two channel cleaning nozzles 73 each have the same configuration, Figs. 16 and 17 will be used to describe in detail one of the channel cleaning nozzles 73. Fig. 17 is a diagram illustrating a cross-section of this channel cleaning nozzle 73. The positions of the channel cleaning nozzle 73 illustrated in Figs. 16 and 17 are the first retreating position and the second retreating position of the endoscope washer device 40'.

As illustrated in Fig. 16 and Fig. 17, the channel cleaning nozzle 73 is an approximately L-shaped channel that has two flanges 73a and 80 that are provided at a predetermined distance in the lengthwise direction. The channel cleaning nozzle 73 has an O-ring 75 on the periphery of the tip portion, and the proximal-end portion on the lengthwise axis is connected to a solenoid shaft 77a. Further, the channel cleaning nozzle 73 passes through a side wall portion of the cleaning sink 2', between the two flanges 73a and 80.

Of the two flanges 73a and 80, the flange 80 on the tip side has a flange face that corresponds to and makes direct contact with one face of a stopper 82 that is a plate member in an L-shape that is fixed on the floor face of the cleaning sink 2'. Also, the other flange 73a on the proximal-end side has a face that makes contact with one end of a later-described spring 81.

The channel cleaning nozzle 73 is configured with a spring 81 between the sealing member 74 of the cleaning sink 2' and the flange 73a. Due to the spring action of this spring 81, the channel cleaning nozzle 73 is attached to the exterior of the cleaning sink 2', and thus one portion of the flange face of the flange 80 of the channel cleaning nozzle 73 makes contact with one portion of one face of the stopper 82 in a normal state.

In this state, as illustrated in Fig. 17, the length of the channel cleaning nozzle 73 from the flange 80 to the tip is the length wherein the opening portion of the air and water sending channel 70 of the endoscope 1A' to be set in the cleaning sink 2' and the channel cleaning nozzle 73 are separated.

In other words, in the state described above, the stopper 82 provided in the cleaning sink 2' is positioned on the floor face of the cleaning sink 2' and fixed, so that the opening portion of the air and water sending channel 70 of the endoscope 1A' that is set in the cleaning sink 2' and the tip of the channel cleaning nozzle 73 are separated.

Further, the stopper 82 is positioned so as to become approximately parallel with the face of the stopper 82 that corresponds with the side face of the operating unit 37' that has the opening portions of the air and water sending channel 70 and the suction channel 71 of the endoscope 1A' that is set in the cleaning sink 2'. Further, the channel cleaning nozzle 73 is moved towards the endoscope 1A' by the drive mechanism 26 to be described below, but the channel cleaning nozzle 73 has a length wherein the tip portion thereof makes a direct contact with the opening portion of the air and water sending channel 70.

A fluid sending tube 76 is connected to the opening portion of the proximal end of the channel cleaning nozzle 73. This fluid sending tube 76 communicates to the branch channel 27 of the endoscope washer device 40 of the first embodiment. With the endoscope washer device 40' of the present embodiment, the branch channel 27 is a two-legged channel.

The solenoid shaft 77a that is connected to the proximal-end portion of the channel cleaning nozzle 73 has the same axis as the lengthwise axis of the channel cleaning nozzle 73. This solenoid shaft 77a passes through a solenoid main body 77 that is electrically connected to the control circuit 200, and comprises a drive mechanism 26 by the solenoid shaft 77a and the solenoid main body 77.

This solenoid main body 77 is fixed to an affixing plate 78 that is fixed to the frame (not illustrated) of the endoscope washer device 40'.

Next, the operation of the endoscope washer device 40' relating to the present embodiment will be described. Further, with this operation description of the endoscope washer device 40', of the two channel cleaning nozzles 73, the channel cleaning nozzle 73 on the side of the air and water sending channel 70 will be described, and the channel cleaning nozzle 73 on the side of the suction channel 71 has the same configuration and operation, and therefore the description thereof will be omitted.

As with the endoscope washer device 40 of the first embodiment, the worker first opens the top cover 3' of the endoscope washer device 40' of the present embodiment, and sets the endoscope 1A' into the cleaning sink 2' inside the endoscope washer device 40.

In this instance, the worker sets the operating unit 37' of the endoscope 1A' in the predetermined position in the first holding member 4' of the cleaning sink 2' so that the side face that has the opening portion such as the air and water sending channel 70 of the operating unit 37' faces the channel cleaning nozzle 73. Further, the worker sets the universal cord 1c' and the insertion portion 1' of the endoscope 1A' while selecting the multiple second holding members 5'.

In the instance of setting the universal cord 1c' and the insertion portion 1' of the endoscope 1A' into the second holding members 5', the worker should set as if drawing a smooth curved line with the bending portion of the insertion portion 1'.

Then the detecting sensor of the first holding member 4', here an optical sensor, detects that the operating unit 37' of the endoscope 1A' is set in the predetermined direction, and supplies a detection signal to the control circuit 200'. The control circuit 200' that receives this detection signal supplies a drive signal to the drive mechanism 26', and the solenoid main unit 77 of the drive mechanism 26' pushes the solenoid shaft 77a to the side of the channel cleaning nozzle 73.

Therefore, as illustrated in Fig. 18, the channel cleaning nozzle 73 that is connected to the solenoid shaft 77a protrudes toward the air and water sending channel 70 of the operating unit 37' of the endoscope 1A'.

Further, the O-ring 75 provided on the tip portion of the channel cleaning nozzle 73 makes direct contact with the opening portion of the air and water sending channel 70, and the channel cleaning nozzle 73 and the air and water sending channel 70 are connected. The position wherein these channel cleaning nozzles 73 are connected to the air and water sending channel 70 of the endoscope 1A' serves as the first use position of the endoscope washer device 40'.

Further, the worker injects a predetermined amount of liquid detergent into the sink of the cleaning sink 2' so as to not directly touch the endoscope 1A'.

Next, the worker closes the top cover 3', presses the start switch that is on the operating panel 41' of the endoscope washer device 40', and operates the endoscope washer device 40'.

Following is a detailed description of the cleaning process of the endoscope washer device 40'.

The endoscope washer device 40' begins the cleaning process of the endoscope 1A' when the start switch on the operating panel 41' is pressed. First, the water supply valve 17' of the endoscope washer device 40' opens, and a cleaning fluid, in this case tap water, from the nozzle 18' is supplied inside the sink of the cleaning sink 2'.

The water supply continues until the water level of the tap water makes contact with one of the liquid face detecting units of the water level detecting sensor 6' on the wall face of the cleaning sink 2'. When the tap water makes contact with the first liquid face detecting unit of the water level detecting sensor 6', the electromagnetic opening/closing valve 25' opens an inner valve, and drives the channel interior cleaning pump 21'. At this time, the tap water continues to be supplied to the inside of the cleaning sink 2' from the nozzle 18'.

Therefore, the cleaning fluid that is stored in the cleaning sink 2', wherein the detergent and the tap water are mixed, passes through the channel interior cleaning channel 19' that communicates to the circulating fluid cleaning/sterilizing channel 9' from the circulating fluid suction opening 20', and is suctioned by the channel interior cleaning pump 21'. The channel interior cleaning pump 21' sends the suctioned cleaning fluid towards the check valve 36' of the channel interior cleaning channel 19'.

The sent cleaning fluid passes through the channel interior cleaning channel 19', passes by the check valve 36', and is sent to the branch channel 27' that communicates to the channel interior cleaning channel 19', and arrives at the electromagnetic opening/closing valve 25'. The cleaning fluid is divided into two channels by the branch channel 27', and then passes through the electromagnetic opening/closing valves 24a' through 24b' and the fluid sending tubes 76, and is sprayed at a predetermined flow rate from the tip opening of the channel cleaning nozzles 73, to the side face that has the air and water sending channel 70 of the operating unit 37' of the endoscope 1A'.

Next, when the liquid face of the cleaning fluid that is supplied into the cleaning sink 2' makes contact with the second liquid face detecting unit of the water level detecting sensor 6', the water supply from the nozzle 18' into the cleaning sink 2' is stopped. Further, the second liquid face detecting unit of the water level detecting sensor 6' is installed at a height from the floor face of the cleaning sink 2' at which the endoscope 1A' will sufficiently be submerged under water. In other words, the endoscope 1A' that is set in the cleaning sink 2' will sufficiently be covered by cleaning fluid.

Next, the circulating fluid cleaning/sterilizing pump 8' starts to drive, and the cleaning fluid within the cleaning sink 2' passes from the circulating fluid suction opening 20' and through two paths, the first circulating fluid path and the second circulating path, and is discharged into the cleaning sink 2'. Of these two paths, the first circulating fluid path is the path wherein the cleaning fluid that is suctioned by the circulating fluid suction opening 20' discharged toward the endoscope 1A' from the channel cleaning nozzle 73, by the above described channel interior cleaning pump 21. The second circulating fluid path is the same path as that of the endoscope washer device 40 of the first embodiment, and therefore the description thereof will be omitted.

The cleaning fluid that circulates through the first circulating fluid path performs cleaning primarily on the end face of the operating unit 37' of the endoscope 1A' and on the various channels that pass through the inside of the endoscope 1A'. Further, the cleaning fluid that circulates through the second circulating fluid path performs cleaning primarily on the exterior surface of the endoscope 1A' by the circulating fluid discharge opening 7'.

The cleaning process of the endoscope washer device 40' is performed for a predetermined length of time, and during this cleaning process, the channel cleaning nozzle 73 makes at least one advancing movement and retreating movement toward and away from the endoscope 1A'. Specifically, the tip portion of the channel cleaning nozzle 73 illustrated in Fig. 18 reciprocates at least once during the cleaning process of the endoscope washer device 40' between the position that is inserted into the air and water sending channel 70 of the operating unit 37' of the endoscope 1A' and the position wherein the channel cleaning nozzle 73 is separated from the opening of the air and water sending channel 70.

To describe more specifically, when the current from the control circuit 200' to the solenoid main body 77 is stopped, the channel cleaning nozzle 73 is moved toward the right side in Fig. 18 by the spring action of a spring 81, and makes contact with one portion of the face of a stopper 82 that corresponds to one portion of the flange face of a flange 80, and stops.

Then after a predetermined length of time passes, the control circuit 200' supplies current again to the solenoid main body 77 that is the drive mechanism 26', and the solenoid main body 77 pushes out the solenoid shaft 77a towards the left in Fig. 17.

Therefore, the channel cleaning nozzle 73 that connects to the solenoid shaft 77a is pushed out towards the opening of the air and water sending channel 70 of the endoscope 1A', and as Fig. 18 illustrates, one portion of the tip portion thereof is inserted into the air and water sending channel 70. These movements are performed at least once during the execution of the cleaning process of the endoscope 1A'.

With the position of the channel cleaning nozzle 73 illustrated in Fig. 18, since the O-ring 75 of the channel cleaning nozzle 73 makes direct contact with the opening end of the air and water sending channel 70 of the endoscope 1A', the flow of the cleaning fluid passes inside the air and water sending channel 70 of the endoscope 1A' and flows out from the tip of the insertion portion 1' into the cleaning sink 2'.

Therefore, the cleaning fluid that circulates through first path performs cleaning primarily on the air and water sending channel 70 that passes through the inside of the endoscope 1A' toward the endoscope 1A' of the channel cleaning nozzle 73, by the reciprocating movement in two directions, and performs cleaning on the side face that has the air and water sending channel 70 of the operating unit 37'.

Further, during the predetermined operation of the endoscope washer device 40, the flow rate sensor of the electromagnetic opening/closing valves 24a' through 24b' constantly measure the flow rate of the cleaning fluid that passed through the inside of the electromagnetic opening/closing valves 24a' through 24b'. In the case that the flow rate passing thorough the inside of the electromagnetic opening/closing valves 24a' through 24b' is lower than that predetermined, the flow rate sensor supplies a warning signal to the control circuit 200', and the control circuit 200' instructs the operating panel 41' to display a warning and the buzzer to sound a warning sound. When the warning signal has been supplied to the control circuit 200' for a predetermined length of time, the control circuit 200' supplies a stop signal to the various equipment of the endoscope washer device 40', and stops the various drives.

After the predetermined length of cleaning processing time has passed, the drives of the circulating fluid cleaning/sterilizing pump 8' and the channel interior cleaning pump 21' are stopped. Further, the switching valve 13' opens the inner valve to the side of the draining channel 35', and enables the cleaning sink 2' and the draining channel 35' to communicate. Simultaneous with the operation of this switching valve 13', the draining pump 28' begins to drive, and sends the cleaning fluid within the cleaning sink 2' from the draining channel 35' on the side of the cleaning sink 2' into the drain hose 42'.

Therefore, the cleaning fluid that is stored within the cleaning sink 2' is discharged to the outside of the endoscope washer device 40'. In order for the cleaning fluid to be sufficiently discharged, after the draining pump 28' drives for a predetermined length of time, the draining pump 28' stops driving, and the switching valve 13' closes the inner valve.

Thus, the cleaning process of the endoscope washer device 40' is completed, and is followed by the start of the rinsing process that rinses the endoscope 1A'.

The rinsing process of the endoscope washer device 40' will be described below, however, the operations similar to the cleaning process will be described in an abbreviated manner.

First, tap water from the spigot 16' is supplied into the sink of the cleaning sink 2' from the nozzle 18'. Similar to the cleaning process, when the tap water makes contact with the first liquid detecting unit of the water level detecting sensor 6', the valve inside the electromagnetic opening/closing valve 25' opens, and the channel interior cleaning pump 21' begins to drive. At this time, the tap water continues to be supplied from the nozzle 18' inside the cleaning sink 2'.

Since the valve of the electromagnetic opening/closing valve 25' is open, the rinse water that is tap water is divided into two channels by the branch channel 27', and a predetermined flow rate is sprayed from the channel cleaning nozzle 73 onto a side face that has an opening such as the air and water sending channel 70 of the operating unit 37' of the endoscope 1A'.

While the endoscope washer device 40' continues the above-described operation, when the liquid face of the tap water makes contact with the second liquid face detecting unit of the water level sensor 6', the water supply from the nozzle 18' into the cleaning sink 2' is stopped. At this time, the endoscope 1A' that is set inside the cleaning sink 2' is sufficiently submerged under water.

Next, the circulating fluid cleaning/sterilizing pump 8' begins to drive, and the channel interior cleaning pump 21' also continues to drive, and the rinse water inside the cleaning sink 2' circulates from the circulating fluid suction opening 20' through the first circulating fluid path and the second circulating fluid path.

The rinsing process of the endoscope washer device 40' is performed for a predetermined length of time, and during the execution of this rinsing process, the channel joint portion 22' makes at least one advancing movement and retreating movement toward and away from the endoscope 1A'.

Therefore, rinse water that circulates through the first path primarily rinses the air and water sending channel 70 that passes through the inside of the endoscope 1A', and rinses a side face that has the opening portion of the air and water sending channel 70 of the operating unit 37', by the movement in two back and forth directions toward the endoscope 1A' of the channel cleaning nozzle 73.

Further, while the rinse water is circulating through the first circulating fluid path, the flow rate sensor of the electromagnetic opening/closing valves 24a' through 24b' constantly measure the flow rate of the rinse water that passes through the inside of the electromagnetic opening/closing valves 24a' through 24b'. In the case that the flow rate of rinse water passes through the inside of the electromagnetic opening/closing valves 24a' through 24b' is less than that predetermined, the control circuit 200' sends instructions to the operating panel 41' to display a warning and to the buzzer to sound a warning sound. When the warning signal is continued for a predetermined length of time, the various equipment of the endoscope washer device 40' stops operating.

When the predetermined length of rinsing process time has passed, the circulating fluid cleaning/sterilizing pump 8' and the channel interior cleaning pump stop driving. Further, the switching valve 13' opens the valve therein to the side of the draining channel 35', and enables the cleaning sink 2' and the draining channel 35' to communicate, and the draining pump 28' begins to drive.

Therefore, the rinse water that is stored in the cleaning sink 2' is discharged to the outside of the endoscope washer device 40'. In order for the rinse water to be sufficiently discharged, the draining pump 28' stops driving after a predetermined length of time.

At this time, the tip portion of the channel cleaning nozzle 73 stops at the position in the state illustrated in Fig. 18 wherein the tip portion of the channel cleaning nozzle 73 is inserted into the opening portion of the air and water sending channel 70 of the endoscope 1A'.

Next, the compressor 31' begins to drive, and supplies compressed air to the air supply channel 30'. This compressed air passes through the channel cleaning nozzle 73, and is blown into the air and water sending channel 70 of the endoscope 1A'.

Further, the compressed air that is blown into the air and water sending channel 70 sends the remaining water inside the air and water sending channel 70 toward the tip of the insertion portion 1' of the endoscope 1A', and blows out from the opening at the tip of the air and water sending channel 70.

Therefore, the remaining water inside the air and water sending channel 70 flows out into the sink of the cleaning sink 2' from the opening in the tip of the air and water sending channel 70, and the inside of the air and water sending channel 70 is dried.

After the above described rinsing process is completed, the endoscope washer device 40' follows by performing a sterilization process. The sterilizing process of the endoscope washer device 40' will be described below, however, the configurations and operations similar to the cleaning process and rinsing process will be described in an abbreviated manner. Further, until the above-described rinsing process is completed, the switching valve 13' closes off the chemical collecting channel 23' and the draining opening 14' by the inner valve.

Next, the sterilization process of the endoscope washer device 40' will be described in detail.

First, the sterilizing fluid that is stored within the tank of the sterilizing fluid tank 32' is sent to the side of the sterilizing fluid injection channel 33' that is connected to the water supply channel 15'. This sterilizing fluid is sent into the water supply channel 15', and flows into the sink of the cleaning sink 2' from the nozzle 18'.

When the endoscope 1A' that is set in the cleaning sink 2' is sufficiently submerged in the sterilizing fluid, the injecting pump 34' stops, and the channel interior cleaning pump 21' begins to drive. The timing of supplying the stop signal to the injecting pump 34' and the drive signal to the channel interior cleaning pump 21' by the control circuit 200' is the time at which the second liquid face detecting unit of the water level detecting sensor 6' detects the liquid face of the sterilizing fluid.

The channel interior cleaning pump 21' begins to drive, and as with the cleaning process, the sterilizing fluid circulates throughout the first circulating fluid path. Therefore, the sterilizing fluid is discharged into the air and water sending channel 70 of the endoscope 1A' by the channel cleaning nozzle 73. Further, during the execution of the sterilizing process, the channel cleaning nozzle 73 supplies and stops the current from the control circuit 200' to the solenoid main body 77 of the drive mechanism 26', and performs at least one reciprocating advancing movement and retreating movement toward and away from the endoscope 1A'.

Further, during the sterilizing process of the endoscope washer device 40', a drive signal can be supplied from the control circuit 200' to the circulating fluid cleaning/sterilizing pump 8', and the circulating fluid cleaning/sterilizing pump 8' can be driven so that the sterilizing fluid is circulated through the second circulating path.

Therefore, the external surface of the endoscope 1A' is exposed to the sterilizing fluid that is stored inside the sink of the cleaning sink 2', and the operating unit 37' and the air and water sending channel 70 therein are sterilized by the sterilizing fluid that circulate through the first circulating path.

After a predetermined length of time has passed, the switching valve 13' opens the inner valve so as to communicate the chemical collecting channel 23' with the drain opening 14'. Therefore, the sterilizing fluid that is stored in the cleaning sink 2' is collected in the sterilizing fluid tank 32'.

Thus, the endoscope washer device 40' completes the sterilizing process, and begins again the rinsing process described above. This rinsing process of the endoscope washer device 40' is similar to the above described operation, and therefore the description thereof will be omitted.

Further, as described above, while the compressed air from the compressor 31' that is the rinsing process is removing water from the air and water sending channel 70 that pass through the inside of the endoscope 1A' for a predetermined length of time, the draining pump 28' drives, and the rinse water inside the sink of the cleaning sink 2' is discharged outside of the endoscope washer device 40' from the draining hose 42'. With the rinsing process after the sterilization process of the endoscope washer device 40', the draining pump 28' continues driving for a predetermined length of time after the compressor 31' receives a stop signal from the control circuit 200' and stops driving. During the drive of the draining pump 28', the switching valve 13' is open so that the inner valve communicates the draining channel 35' and the drain opening 14'. As a result, approximately all of the rinse water inside the cleaning sink 2' is discharged to the outside of the endoscope washer device 40' from the drain opening 14'.

Lastly, the various electromagnetic valves and so forth that are controlled by the control circuit 200' return to the predetermined beginning set positions, and the endoscope washer device 40' completes the cleaning and sterilization of the endoscope 1A'. After this, the worker opens the top cover 3' of the endoscope washer device 40', separates the operating unit 37' of the endoscope 1A' from the first holding member 4', separates the universal cord 1c from the second holding member 5', and completes the work by removing the endoscope 1A' from the cleaning sink 2' of the endoscope washer device 40'.

As a result of the above, according to the endoscope washer device 40' of the present embodiment, in addition to the advantages of the endoscope washer device 40 according to the first embodiment, an endoscope 1A like that in the first embodiment that corresponds to the endoscope washer device 40' does not need to be purchased, because the tip portions of the channel cleaning nozzles 73 each insert to or detach from the drive mechanism 26' wherein one side face of the operating unit 37' of the existing endoscope 1A' has the air and water sending channel 70 and the suction channel 71.

Further, the operating unit 37' of the endoscope 1A' that is set therein is easily positioned by the three first holding members 4', and because the direction thereof is regulated, the center axis of the opening portion of the air and water sending channel 70 and the suction channel 71 of the operating unit 37' can be positioned along approximately the same axis in the lengthwise axis of the two channel cleaning nozzles 73 that extend from one wall face of the cleaning sink 2'.

Therefore, the two channel cleaning nozzles 73 can be inserted in the opening portions of the air and water sending channel 70 and the suction channel 71 of the endoscope 1A' with certainty.

### [Third Embodiment]

Next, a third embodiment of the endoscope washer device 40' will be described. Now, the description of the endoscope washer device 40' relating to the present embodiment will use the same reference numerals for components with the same configuration as those of the endoscope washer device 40 and 40' of the first embodiment and second embodiment, and description thereof will be omitted. Also, the operations and advantages will also be described in an abbreviated manner.

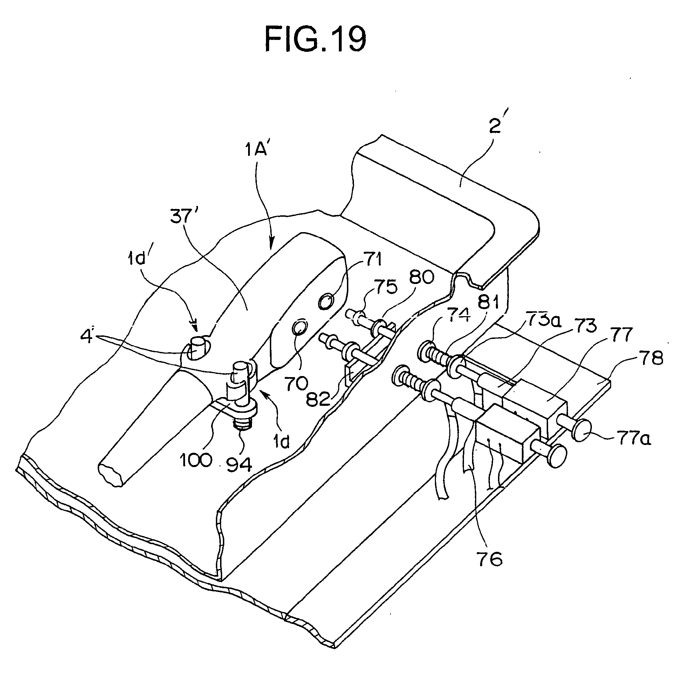

Fig. 19 is a configuration diagram illustrating the inside a portion of the endoscope washer device 40' illustrating an internal portion wherein the endoscope 1A' is set in the sink of the cleaning sink 2' of the endoscope washer device 40' relating to the present embodiment.

The two first holding members 4' that project so as to be perpendicular to the floor face of the cleaning sink 2' of the endoscope washer device 40' illustrated in Fig. 19 are each inserted into the guide holes 1d' of the operating unit 37' of the endoscope 1A'. The endoscope 1A' is positioned and set in the predetermined position within the cleaning sink 2' of the endoscope washer device 40' by these two first holding members 4'. In the state wherein the endoscope 1A' is set, the endoscope 1A' is positioned on approximately the same axis as the lengthwise axis of the two channel cleaning nozzles 73 and the center axes of the opening portions of the air and water sending channel 70 and the suction channel 71 of the operating unit 37' thereof.

Now, since the two channel cleaning nozzles 73 and the surrounding configuration thereof is a configuration identical to that of the second embodiment, description thereof will be omitted.

The two first holding members 4' that protrude from the floor face of the cleaning sink 2' are passed through the holes of a contact 100 that make the two into one unit. A spring 94 is configured on each of the two first holding members 4' between the contact 100 and the floor face of the cleaning sink 2'.

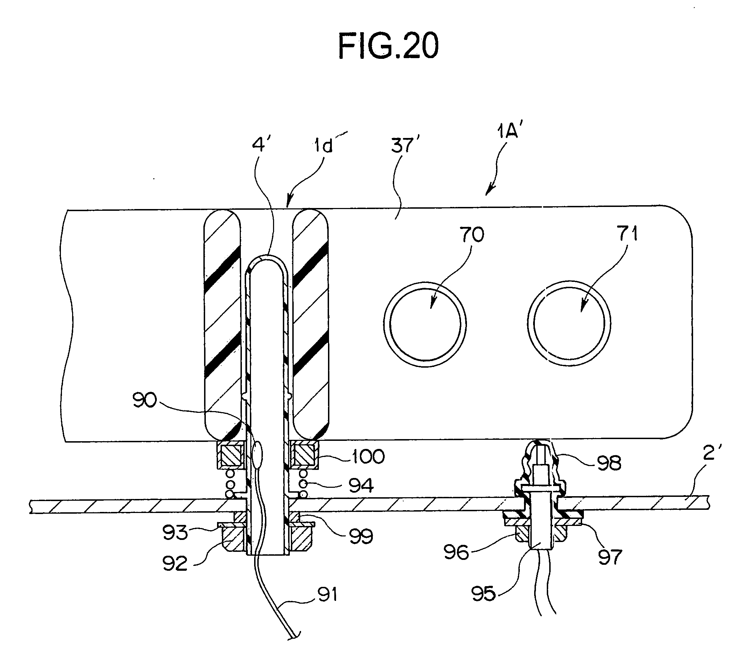

Further, a detecting switch 95 (see Fig. 20) that is covered by a rubber cover 98 makes direct contact with one side face of the operating unit 37' of the endoscope 1A' and protrudes from the floor face of the cleaning sink 2' so as to be approximately perpendicular to the face thereof.

Next, the configuration of the first holding member 4' and the detecting switch 95 will be described with reference to Fig. 20. Fig. 20 is a cross-sectional diagram illustrating the external portion of the cleaning sink 2' wherein the endoscope 1A' is set in the first holding members 4' of the cleaning sink 2' of the endoscope washer device 40'. Further, in the following description, the two first holding members 4' are provided on the cleaning sink 2', but since these have same configurations, only one of the two first holding members 4' will be described, and the description of the other will be omitted.

As illustrated in Fig. 20, the first holding member 4' is pushed through the floor face of the cleaning sink 2' of the endoscope washer device 40' so as to be approximately perpendicular to the face thereof. This first holding member 4 is formed as a cylinder wherein one end is closed, and has a convex portion and a flange in the middle portion thereof.

The convex portion of this first holding member 4' makes direct contact with the hold face of the guide hole 1d' of the operating unit 37' of the endoscope 1A'. Further, the proximal-end portion of the first holding member 4' is passed through the floor face of the cleaning sink 2' of the endoscope washer device 40' on the device inner side. The periphery of the proximal-end portion of the first holding member 4 is fixed here with a fixing nut 92 serving as a fixing member, and a washer 93 that makes contact with this fixing nut 92.

Further, the hole face of a packing 99 that is provided on the cleaning sink 2' adheres to the periphery face of the first holding member 4', and one end face makes contact with the face on the inner side of the device of the cleaning sink 2', and the other end face makes contact with the cleaning sink 2' side of the washer 93, and thus is adhered. Thus, the interior and exterior of the cleaning sink 2' maintains water-tightness. In other words, the first holding member 4' is fixed onto the cleaning sink 2' so that the floor face of the cleaning sink 2' is sandwiched by the packing 99 and the flange of the first holding member 4'.

The spring 94 is fit on the first holding member 4' that protrudes into the sink of the cleaning sink 2', such that one end makes contact with the floor face of the cleaning sink 2', and the first holding member 4' passes through the hole of the contact 100 that has a magnet within, to which the other end of the spring 94 is attached.

The spring 94 normally pushes up the contact 100 in the direction away from the floor face of the cleaning sink 2' when the endoscope 1A' is not set into the cleaning sink 2'. Further, the external diameter of the portion of the above-described first holding member 4' that has the convex portion is larger than the hole diameter of the contact 100, and therefore the contact 100 is prevented from pulling loose from the first holding member 4'.

A lead switch 91 is on the inside of the first holding member 4', and is electrically connected to the control circuit 200' within the endoscope washer device 40 by the lead wire. This lead switch 91 supplies an ON signal to the control circuit 200' when the magnet of the contact 100 moves close to the predetermined distance.

Next, the detecting switch 95 illustrated in Fig. 20 will be described.

The detecting switch 95 passes through the predetermined hole of the cleaning sink 2', and the flange is in direct contact with the floor face of the cleaning sink 2'. Specifically, the hole on the floor face of the cleaning sink 2' is provided within a region of the face that corresponds to the floor face of the cleaning sink 2' of the operating unit 37' of the endoscope 1A', as illustrated in Fig. 20, and the detecting switch 95 protrudes through this hole toward the interior of the cleaning sink 2' in the direction of being approximately perpendicular to the face of the floor face of the cleaning sink 2'.

Further, the proximal-end portion of the detecting switch 95 has a fixing member, for example a fixing nut 96 and a washer 97 between the face of the device interior of the cleaning sink 2' and this fixing nut 96.

This detecting switch 95 is covered by a cylinder with elasticity that here is the rubber cover 98. The tip of this rubber cover 98 is closed, and the inside face of the tip face of the rubber cover 98 and the tip face of the detecting switch 95 correspond with each other. Further, the opening portion of the proximal end of the rubber cover 98 is in the form of a flange, and the flange form portion is sandwiched between the washer 97 of the detecting switch 95 and the cleaning sink 2'.

In other words, the fixing nut 96 of the detecting switch 95 fixes the detecting switch 95 to the cleaning sink 2' so as to adhere the flange-formed portion of the rubber cover 98 to the face of the device interior of the floor face of the cleaning sink 2', and to the face of the washer 97 of the detecting switch 95 that corresponds to the face thereof. Thus, the inside of the sink of the cleaning sink 2' and the device interior maintain water-tightness.

Therefore, the floor face of the cleaning sink 2' is sandwiched by a rubber cover 98 and the detecting switch 95 is fixed to the cleaning sink 2' by the flange of the detecting switch 95 and the washer 97.

Further, the detecting switch 95 is electrically connected to the control circuit 200' within the endoscope washer device 40', and supplies a detecting signal to the control circuit 200'.

Next, the operation of the endoscope washer device 40' of the present embodiment will be described. The operation description of the endoscope washer device 40' regarding the cleaning process, the rinsing process, and the sterilization process is identical to that of the cleaning process, the rinsing process, and the sterilization process of the above described second embodiment, and therefore the description thereof will be omitted.

Further, the various operating switches and bending knob that the operating unit 37' has are not shown for the endoscope 1A' illustrated in Fig. 19 that is set in the cleaning sink 2'.

The worker sets the endoscope 1A' inside the sink of the cleaning sink 2' of the endoscope washer device 40', as illustrated in Fig. 19. In this instance, this inserts the first holding members 4 into the two guide holes 1d of the operating unit 37' of the endoscope 1A'. Then the worker sets the endoscope 1A' so as to push in toward the cleaning sink 2', so that the face that has the opening portion of the air and water sending channel 70 and the suction channel 71 of the operating unit 37' is corresponding with the side wall face of the cleaning sink 2' wherein the channel cleaning nozzles 73 protrude.

When the endoscope 1A' is set, the face of the operating unit 37' of the endoscope 1A' that corresponds to the floor face of the cleaning sink 2' presses the detecting switch 95 and the contact 100 of the first holding member 4' of the endoscope 1A' to the side of the cleaning sink 2'.

Specifically, the face near the guide hole 1d' of the operating unit 37' that corresponds to the floor face of the cleaning sink 2' makes direct contact with the face on the operation unit 37' side of the contact 100, and moves the contact 100 toward the cleaning sink 2' along the lengthwise axis direction of the first holding member 4'.

Therefore, as the magnet inside the contact 100 nears the predetermined distance of the detecting sensor 90 inside the first holding member 4', in other words, as the detecting sensor 90 nears the distance at which it can detect the magnetism of the magnet in the contact 100, the detecting sensor 90 supplies a detecting signal to the lead switch 91. The lead switch 91 then turns ON and supplies an ON signal to the control circuit 200'.

Further, the direct contact portion of the operating unit 37' that directly contacts the tip of the rubber cover 98 that covers the detecting switch 95 elastically changes the shape of the rubber cover 98 so as to shrink in the direction of the cleaning sink 2', and the position of the tip portion of the detecting switch 95 inside the rubber cover 98 is moved to the side of the cleaning sink 2'. Therefore, the detecting switch 95 supplies an ON signal to the control circuit 200' by moving the tip portion thereof.

As a result of the above, when the endoscope 1A' is correctly set inside the cleaning sink 2' of the endoscope washer device 40', the detecting sensor 90 and the detecting switch 95 supply an ON signal to the control circuit 200' from the lead switch 91 and the detecting switch 95, and the control circuit 200' supplies a drive signal to the drive mechanism 26' for the purpose of moving the channel cleaning nozzle 73 to the air and water channel 70 and the suction channel 71 of the endoscope 1A'. Here, the control circuit 200' does not supply a drive signal to the drive mechanism 26' if the two ON signals are not supplied by the lead switch 91 and the detecting switch 95.

Next, when the channel cleaning nozzle 73 is linked with the air and water channel 70 and the suction channel 71 of the endoscope 1A', the endoscope washer device 40' performs a cleaning process, a rinsing process, and a sterilization process, in this order.

In other words, if the endoscope 1A' is not correctly set in the first holding member 4' within the sink of the cleaning sink 2', the endoscope washer device 40' does not start the cleaning process. Now, at this time, a mechanism can be provided to display a warning or generate a warning sound that would alert the worker.

Further, during the various processes of the endoscope washer device 40', in the case that the operating unit 37' of the endoscope 1A' becomes loosened from the first holding member 4', or if it has moved more than a predetermined amount, the detecting sensor 90 and the detecting switch 95 detects the abnormality, and the control circuit 200' is supplied with an OFF signal from the lead switch 91 and the detecting switch 95.

The control circuit 200' then causes the operating panel 41' to display a warning, and along with a warning sound, stops the driving of the endoscope washer device 40'.

As a result of the above, in addition to the advantages of the first embodiment and the second embodiment, the endoscope washer device 40' according to the present embodiment can perform cleaning and sterilization of the endoscope 1A' with certainty and a high degree of safety because the various drives stop in the event that the endoscope 1A' is not correctly set in the cleaning sink 2'.

Further, if both of the detecting sensor 90 and the detecting switch 95 do not detect that the endoscope 1A' has been correctly set in the cleaning sink 2', the endoscope washer device 40' does not perform the next operation and various processes, and therefore cleaning and sterilization of the endoscope 1A' can be performed with greater certainty.

Now, in the case that only one of the detecting sensor 90 or the detecting switch 95 is used, manufacturing costs can be lowered.

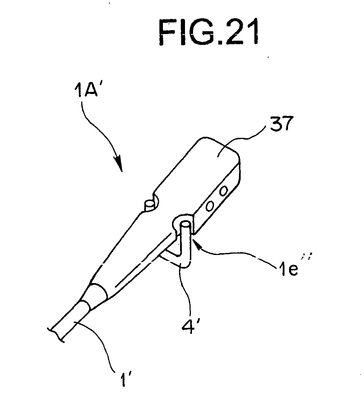

Further, as illustrated in Fig. 21, the endoscope 1A' can be provided with a groove portion such as a positioning groove 1e" on the operating unit 37', similar to the guide groove 1e' that is provided on the fist insertion portion 1a of the insertion portion 1 described in the first embodiment.

Further, the first holding members 4 and 4', and the second holding members 5 and 5' may be formed of a hard material or may be a material with elasticity that has a predetermined rigidity.

### Industrial Applicability

When performing the cleaning or sterilizing of the endoscope, since it is possible to easily set the endoscope in a cleaning sink and reliably perform the cleaning and sterilizing of the various channels of the endoscope without connecting work by the user to connect the multiple cleaning tubes of the endoscope washer device to the various channels within the endoscope, time is saved in connecting the multiple cleaning tubes and time needed for performing cleaning and sterilizing can be reduced, thereby improving the workability of the endoscope.

## Claims

1. An endoscope washer system comprising:
an endoscope having a channel positioned on the inside thereof and comprising an insertion portion and an operating unit; and
an endoscope washer device comprising a fluid supplying channel capable of moving between a retreated position distanced from the channel of the endoscope set in a predetermined position within the cleaning sink, and a first utilizing position, to which the fluid supplying channel advances, toward the channel side from this retreated position, and makes connection to the channel of the endoscope, and a fluid channel drive mechanism that recognizes that the endoscope is positioned at the predetermined position within the cleaning sink, and automatically moves the fluid supplying channel from the retreated position to the first utilizing position.

2. An endoscope washer system comprising:
an endoscope comprising an insertion portion, wherein a channel is positioned from the tip to the proximal end, and an operating unit that is linked to the insertion portion,
wherein the insertion portion is detachable from the operating unit; and
an endoscope washer device comprising a fluid supplying channel capable of moving between a retreated position distanced from the channel of the endoscope set in a predetermined position within the cleaning sink and a first utilizing position, to which the fluid supplying channel advances, toward the channel side from this retreated position, and makes connection to the channel of the endoscope, and a fluid channel drive mechanism that recognizes that the endoscope is positioned at the predetermined position within the cleaning sink, and automatically moves the fluid supplying channel from the retreated position to the first utilizing position.

3. An endoscope washer system according to Claim 1 or 2, wherein, in at least one of a cleaning process, sterilizing process, and rinsing process, the fluid channel drive mechanism retracts the fluid supplying channel that is at the first utilizing position in the direction of the retreated position, and the endoscope channel and the fluid supplying channel form a predetermined spacing, and moves to a second utilizing position wherein the fluid can be supplied at the opening edge of the channel.

4. An endoscope washer system according to Claim 3,
wherein the second utilizing position and the retreated position are the same position.

5. An endoscope washer system according to any one of Claims 1 to 4, wherein the insertion portion has a retaining portion that retains the operating unit or a retained portion that is retained by the operating unit, and is detachable from the operating unit.

6. An endoscope washer system according to any one of Claims 1 to 5, further comprising a holding member for the purpose of holding the endoscope in the cleaning sink,
wherein the endoscope has a guide section held by the holding member for the purpose of being positioned within the cleaning sink.

7. An endoscope washer system according to any one of Claims 1 to 6, wherein the endoscope comprises notification means for the purpose of informing of being held in the holding member as predetermined.

8. An endoscope washer device, comprising:
a fluid supplying channel capable of moving between a retreated position distanced from the channel of the endoscope set in a predetermined position within the cleaning sink and a first utilizing position, to which the fluid supplying channel advances, toward the channel side from this retreated position, and makes connection to the channel of the endoscope; and
a fluid channel drive mechanism that recognizes that the endoscope is positioned at the predetermined position within the cleaning sink, and automatically moves the fluid supplying channel from the retreated position to the first utilizing position.

9. An endoscope washer device according to Claim 8, wherein, in at least one of a cleaning process, sterilizing process, and rinsing process, the fluid channel drive mechanism retracts the fluid supplying channel that is at the first utilizing position in the direction of the retreated position, and the endoscope channel and the fluid supplying channel form a predetermined spacing, and moves to a second utilizing position wherein the fluid can be supplied at the opening edge of the channel.

10. An endoscope washer device according to Claim 9, wherein the second utilizing position and the retreated position are the same position.

11. An endoscope comprising:
an insertion portion, wherein an endoscope channel is positioned from the tip to the proximal end; and
an operating unit that is linked to the insertion portion;
wherein the insertion portion is detachable from the operating unit.

12. An endoscope according to Claim 11, wherein the insertion portion has a retaining portion that retains the operating unit or a retained portion that is retained by the operating unit, and is detachable from the operating unit.

13. An endoscope held by a holding member that is provided in a cleaning sink of an endoscope washer device and subjected to cleaning and sterilizing, the endoscope comprising:
an insertion portion, wherein an endoscope channel is positioned from the tip to the proximal end; and
an operating unit that is linked to the insertion portion,
wherein the insertion portion has a guide section held by the holding member for the purpose of being positioned within the cleaning sink.

14. An endoscope according to Claim 13, wherein the insertion portion further has notification means for the purpose of informing of being held in the holding member as predetermined.
